# EUROPÄISCHE PATENTSCHRIFT

(11) **EP 2 803 666 B1**
(45) Veröffentlichungstag und Bekanntmachung des Hinweises auf die Patenterteilung: **27.04.2016**
(21) Anmeldenummer: 13168230.4
(22) Anmeldetag: 17.05.2013
(51) Int. Cl.: A61K 8/49, A61Q 5/02, A61Q 19/10, C11B 9/00

(54) **Cyclische Acetale und Ketale sowie deren Verwendung als Riechstoff**
Cyclic acetals and ketals and their use as fragrance compounds
Acétals et cétals cycliques et leur utilisation en tant que parfum

(43) Veröffentlichungstag der Anmeldung: 19.11.2014
(73) Patentinhaber: Symrise AG, 37603 Holzminden (DE)
(72) Erfinder: Hölscher, Bernd, 37620 Halle (DE)
(74) Vertreter: Fabry, Bernd

(56) Entgegenhaltungen:
- EP-A1- 0 221 444
- EP-A1- 0 353 670
- EP-A1- 0 761 664
- EP-A1- 1 496 055
- EP-A1- 1 679 311
- EP-A1- 2 287 158
- EP-A1- 2 524 959
- EP-A2- 0 266 648
- DE-A1- 3 603 661
- GB-A- 860 498
- US-A1- 2007 010 542
- DATABASE HCAPLUS [Online] ACS; XP002716032, gefunden im STN Database accession no. 147:349982(DN) & XU, RUOFEI ET AL.: JINGXI HUAGONG, Bd. 23, Nr. 11, 2006, Seiten 1089-1093, ISSN: 1003-5214
- DATABASE HCAPLUS [Online] ACS; XP002716033, gefunden im STN Database accession no. 155:681848(DN) & YANG, XI ET AL.: ZHONGGUO NIANGZAO, Nr. 9, 2010, Seiten 149-151, ISSN: 0254-5071
- PETERS J A ET AL: "Synthesis and conformation of some 2,4-dioxa- and 2,4-dioxa-3-silabicyclo[3.3.1]nonanes", TETRAHEDRON, ELSEVIER SCIENCE PUBLISHERS, AMSTERDAM, NL, Bd. 38, Nr. 24, 1982, Seiten 3641-3647, XP008165695, ISSN: 0040-4020
- MARK J BROWN ET AL: "Acid-promoted reaction of cyclic allylic diols with carbonyl compounds. Stereoselective ring-enlarging tetrahydrofuran annulations", JOURNAL OF THE AMERICAN CHEMICAL SOCIETY, Bd. 113, Nr. 14, 1991, Seiten 5365-5378, XP008165684, ISSN: 0002-7863, DOI: 10.1021/JA00014A031 [gefunden am 1991-07-01]

## Beschreibung

In einem ersten Aspekt betrifft die vorliegende Erfindung Verbindungen der nachfolgend definierten Formel (I) sowie insbesondere deren Verwendung als Riechstoff. In einem weiteren Aspekt betrifft die vorliegende Erfindung Zusammensetzungen und Riechstoffmischungen enthaltend Verbindungen der nachfolgend definierten Formel (I) sowie die mit diesen Zusammensetzungen und Riechstoffmischungen parfümierten Produkte.

Ferner betrifft die vorliegende Erfindung ein Verfahren zum Verstärken eines Geruchs oder mehrerer Gerüche ausgewählt aus der Gruppe bestehend aus fruchtig, blumig, würzig, holzig, moschus und ambriert sowie ein Verfahren zum Versehen von Haaren oder textilen Fasern mit einer, mehrerer oder sämtlichen der Noten ausgewählt aus der Gruppe bestehend aus fruchtig, blumig, würzig, holzig, moschus und ambriert unter Verwendung einer oder mehrerer Verbindungen der nachfolgend definierten Formel (I).

Wegen der im Allgemeinen unzureichenden Verfügbarkeit vieler natürlicher Riechstoffkomponenten, der notwendigen Anpassung an wechselnde modische Geschmacksrichtungen sowie dem ständig steigenden Bedarf an neuen Riechstoffen, die allein oder in Form von Kompositionen wertvolle Duftstoffe bzw. Parfüms mit interessanten Duftnoten darstellen, besteht auch weiterhin ein Bedürfnis nach Verbindungen mit interessanten Riechstoffqualitäten.

Darüber hinaus besteht in der Parfümindustrie wegen der steigenden Nachfrage der Verbraucher nach neuen Duftnoten ein ständiger Bedarf an Riechstoffen, mit denen sich in Parfüms neuartige Effekte erzielen und auf diese Art neue Modetrends kreieren lassen.

Trotz einer Vielzahl bereits vorhandener Riechstoffe besteht in der Parfümindustrie auch weiterhin ein genereller Bedarf an neuen Riechstoffen, insbesondere an solchen, die über Ihre primären, nämlich geruchlichen, Eigenschaften hinaus zusätzliche positive Sekundäreigenschaften besitzen, wie z.B. eine höhere Stabilität unter bestimmten Anwendungsbedingungen, eine hohe Ausgiebigkeit, eine große Strahlungskraft, gute Diffusivität (d.h. eine gute Raumwirkung), Fülle, Kraft und/oder Natürlichkeit, geruchsverstärkende Eigenschaften oder aber auch bessere dermatologische Eigenschaften gegenüber Riechstoffen mit vergleichbaren primären geruchlichen Eigenschaften.

Es besteht daher in der Parfümindustrie grundsätzlich ein Bedarf an weiteren Riechstoffen, die sich zur Herstellung von Riechstoffkompositionen oder parfümierten Artikeln eignen. Insbesondere besteht ein Bedarf an Riechstoffen, die durch die oben erwähnten technischen Eigenschaften zu einem erhöhten Nutzen von Riechstoffkompositionen und Parfümölen führen.

Cedran-Derivate der Formel (V) werden in EP 857 723 A1 und in EP 2 287158 A1 beschrieben. Danach besitzen Verbindungen dieser Stoffklasse einen Geruch vom Ambratyp und vermitteln gleichzeitig einen strahlenden, starken Effekt, der ganz unterschiedliche Parfümnoten verstärkt und ihre Duftwirkung verlängert.

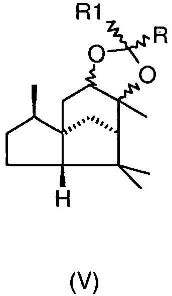

Eine kommerziell erhältliche Verbindung der Formel (V) ist die Verbindung [(4aR,5R,7aS,9R)-Octahydro-2,2,5,8,8,9a-hexamethyl-4H-4a,9-Methanoazuleno(5,6-d)-1,3-dioxol, CAS Nr. 211299-54-6; nachfolgend: Ambrocenide^{®}], welche ebenfalls in EP 0 857 723 A1 beschrieben ist. Ihre Struktur entspricht der nachfolgenden Formel (V'):

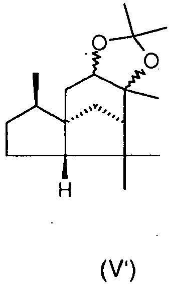

Die geschlängelten Linien in Formel (V') weisen auf die mögliche alpha- und beta-Konfiguration der Verbindung hin. D.h., Ambrocenide^{®} der Formel (V') kann ein, zwei, drei oder sämtliche der folgenden Diastereoisomere umfassen:

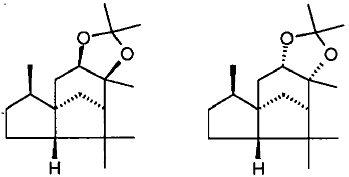

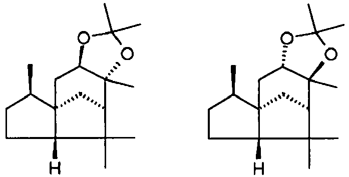

Die Folgenden Schriften offenbaren Verbindungen, die ähnlich zu den Verbindungen der vorliegenden Anmeldung sind, jedoch nicht unter den Schutzbereich der Formel (I) der vorliegenden Anmeldung fallen

In EP 0 761 664 A1 wird die Synthese von Campher-Derivaten der unten stehende Formel und ihre Anwendung als Riechstoffe offenbart.

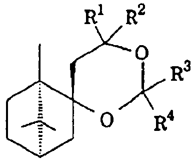

Die EP 0 266 648 A2 offenbart Verbindungen der allgemeinen Formel:

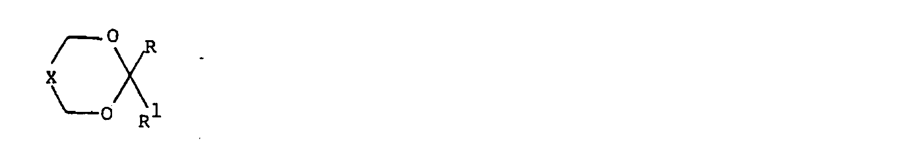

In der EP 1 496 055 A1 wird die Synthese und Anwendung als Riechstoff von 4H-indeno[4,5-D]-1,3-dioxol, 3A,5,6,7,8,8B-hexahydro-2,2,6,6,7,8,8-heptamethyl beschrieben. 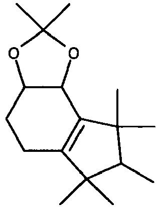 4H-indeno[4,5-D]-1,3-dioxol, 3A,5,6,7,8,8B-hexahydro-2,2,6,6,7,8,8-heptamethyl

Für die Verwendung einer Substanz als Riechstoff sind neben einem interessanten Geruchsprofil aber auch noch andere Eigenschaften wichtig, wie z.B. die Stabilität, die Kompatibilität mit anderen Riechstoffen, die Löslichkeit, sowie die toxikologische Unbedenklichkeit.

Weitere wichtige Eigenschaften sind ferner das Haftvermögen bzw. die Substantivität eines Riechstoffes, insbesondere auf Fasern und/oder Haaren. Von besonderer Bedeutung ist diese Eigenschaft für tensidhaltige Produkte wie Shampoos, Waschmittel und Weichspüler.

Für die Parfümierung von tensidhaltigen Formulierungen von besonderem Interesse sind Ambra-Riechstoffe mit einer starken Eigenhaftung. In diesem Zusammenhang besonders gefragt ist die Geruchsnote von weißer Ambra ("white amber"). Unter "white amber" versteht man parfümistisch den Geruch von gealterter natürlicher Ambra, die eine sehr wertvolle Geruchsnote darstellt. Außerdem sollen die Ambra-Riechstoffe eine gute Bioabbaubarkeit bzw. Bioakkumulation aufweisen.

Eine primäre Aufgabe der vorliegenden Erfindung ist es daher, Verbindungen bereitzustellen, die möglichst viele der zuvor definierten Anforderungen erfüllen.

Insbesondere ist es Aufgabe der vorliegenden Erfindung, eine Substanz (Mischung oder Einzelverbindung) mit Ambra-Geruch und besonders starker Eigenhaftung bereitzustellen, die vorzugsweise eine Geruchsnote von weißer Ambra hat und zusätzlich ein verbessertes toxikologisches Profil aufweist.

Diese Aufgaben werden erfindungsgemäß gelöst durch 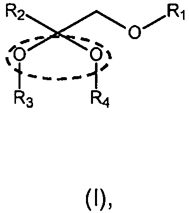 wobei die Verbindung ausgewählt ist aus der
Verbindung der Formel (II) oder (II') 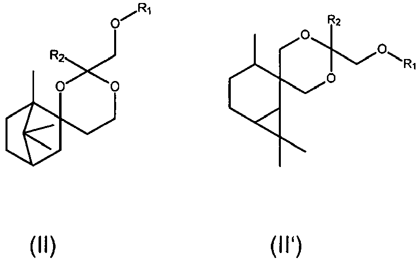 wobei R₁ Wasserstoff oder C₁-C₃-Alkyl, vorzugsweise Wasserstoff, Methyl oder Ethyl, und R₂ Wasserstoff oder C₁-C₄-Alkyl, vorzugsweise Wasserstoff, Methyl oder Ethyl, ist bzw. sind, bevorzugt wobei R₁ und/oder R₂ Methyl oder R₁ Methyl und R₂ Wasserstoff ist bzw. sind oder der Verbindung
der Formel (III) 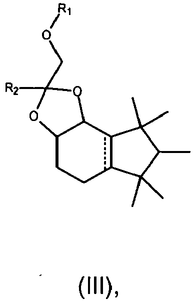 wobei R₁ Wasserstoff oder C₁-C₃-Alkyl, vorzugsweise Wasserstoff, Methyl oder Ethyl, und R₂ Wasserstoff oder C₁-C₄-Alkyl, vorzugsweise Wasserstoff, Methyl oder Ethyl, ist bzw. sind, bevorzugt wobei R₁ und/oder R₂ Methyl oder R₁ Methyl und R₂ Wasserstoff ist bzw. sind, und die Verbindung gesättigt oder ungesättigt ist oder der Verbindung
der Formel (IV) oder (IV') 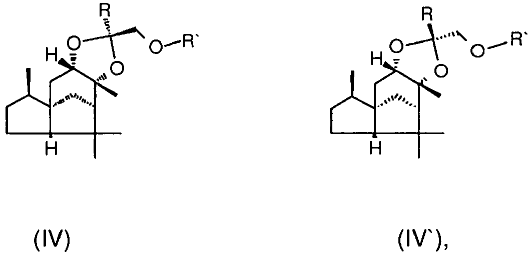 wobei gilt: R = H oder C₁-C₄-Alkyl und R'= H oder C₁-C₃-Alkyl, vorzugsweise R = R' = C₁-Alkyl (im Übrigen gilt für bevorzugte Reste das oben Gesagte entsprechend).

Von der oben angegebenen Formel (II) umfasst sind auch alle Stereoisomere, die sich durch asymmetrische Substitution des zentralen Kohlenstoffatoms ergeben. D.h., die hierin beschriebenen Verbindungen der Formel (II) können jeweils in Form eines bestimmten Stereosimers oder in Form einer beliebigen Mischung verschiedener Stereoisomere vorliegen.

In der oben angegebenen Formel (III) deutet die gepunktete Linie an, dass die Verbindung gesättigt oder ungesättigt sein kann. Ist die Verbindung gesättigt, sind Fünf- und Sechsring über eine gemeinsame Einfachbindung verknüpft; ist die Verbindung ungesättigt, sind Fünf- und Sechsring über eine gemeinsame Doppelbindung verknüpft.

Insbesondere das Stereoisomer der Formel (IV) zeichnet sich durch einen starken Geruch nach Ambra und Holz aus und verfügt vorteilhafterweise über eine bessere Bioabbaubarkeit bzw. bessere Bioakkumulation gegenüber in EP 857 723 A1 und EP 2 287158 A1 genannten Verbindungen. Generell ist an dieser Stelle anzumerken, dass sämtliche der hierin genannten Vorteile erfindungsgemäßer Verbindungen grundsätzlich für die Gesamtheit der hierin beschriebenen erfindungsgemäßen Verbindungen gilt, ganz besonders aber für die hierin als bevorzugt bezeichneten Verbindungen.

Bei der geruchlichen Evaluierung erfindungsgemäßer Verbindungen (wie hierin beschrieben) erwiesen sich diese, insbesondere solche wie oben als bevorzugt beschrieben, nicht nur als geruchlich interessant. Es zeigte sich ferner, dass diese Verbindungen über eine außergewöhnlich lange Haftung verfügt, die sogar die von Ambrocenide^{®} (EP 857 723 A1) übertrifft.

Die erfindungsgemäßen Verbindungen der Formeln VIII und IX können beispielsweise mittels Umsetzung von cis-Cedrandiol (Verbindung der Formel (VI), welche aus α-Cedren herstellbar ist) mit 1-Methoxy-propan-2-on (Verbindung der Formel (VII)) hergestellt werden.

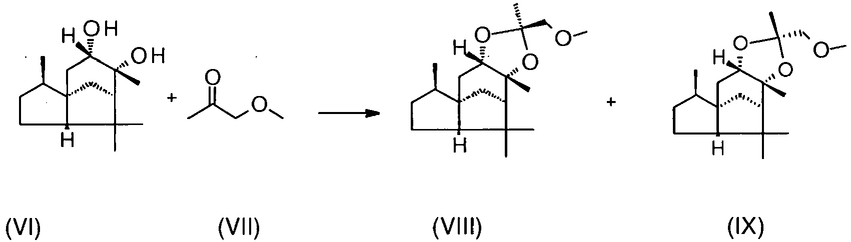

In analoger Weise können durch Umsetzung von cis-Cedrandiol mit Methoxyacetaldehyddimethylacetal die erfindungsgemäßen Verbindungen der Formeln (X) und (XI) erhalten werden.

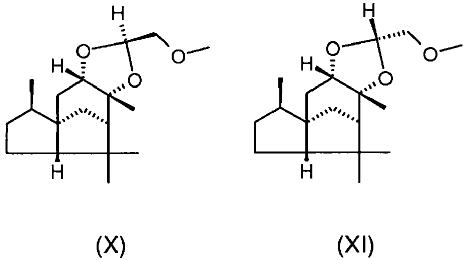

Bevorzugt werden die erfindungsgemäßen Verbindungen der Formeln VIII und IX bzw. X und XI hergestellt durch Umsetzung von cis-Cedrandiol (Formel VI) mit 1-Methoxy-propan-2-on oder Methoxyacetaldehyddimethylacetal in Gegenwart einer oder mehrerer wasserbindender Substanzen und einer oder mehrerer Säuren.

Dementsprechend betrifft die vorliegende Erfindung auch entsprechende Herstellverfahren (wie oben beschrieben). Insbesondere betrifft die vorliegende Erfindung ein Verfahren zur Herstellung erfindungsgemäß besonders bevorzugter Verbindungen der Formel (I) (siehe oben, Verbindungen der Formeln (VIII) bis (XI)) mit folgendem Schritt: Umsetzen einer Verbindung der Formel (VI) (wie oben gezeigt) mit einer Verbindung der Formel (VII) (wie oben gezeigt) oder mit Methoxyacetaldehyddimethylacetal, vorzugsweise in Gegenwart einer oder mehrerer wasserbindender Substanzen und einer oder mehrerer Säuren. In entsprechender Weise lassen sich auch weitere erfindungsgemäße Verbindungen (wie hierin beschrieben) herstellen.

Bevorzugte wasserbindende Substanzen sind ausgewählt aus der Gruppe bestehend aus Orthoameisensäureestern (vorzugsweise Orthoameisensäuretrimethylester oder Orthoameisensäuretriethylester) und 2,2-Dialkoxypentanen (vorzugsweise 2,2-Dimethoxypentan oder 2,2-Diethoxypentan).

Die gegebenenfalls eingesetzten Säuren sind vorzugsweise ausgewählt aus der Gruppe bestehend aus anorganischen Protonensäuren, organischen Protonensäuren und sauren Festbettkatalysatoren.

Vorzugsweise erfolgt die Umsetzung von cis-Cedrandiol (Formel (VI) mit 1-Methoxy-propan-2-on bzw. Methoxyacetaldehyddimethylacetal zu den erfindungsgemäßen Verbindungen der Formeln VIII und IX bzw. X und XI bei einer Reaktionstemperatur im Bereich von 0 bis 70°C.

Die Verbindungen der Formeln (VIII) bis (XI) sind für die Zwecke der hierin beschriebenen Erfindung besonders bevorzugt.

Ein weiterer Aspekt der vorliegenden Erfindung betrifft Zusammensetzungen umfassend oder bestehend aus
(i) ein(em) oder mehrere(n) 3,6,8,8-Tetramethyl-octahydro-3a,7-methano-azulen-5,6-diolketal(en) und
(ii) eine(r) oder mehrere(n) Verbindung(en) der Formel (11), (II'),(III), (IV) und/oder (IV'), bevorzugt eine(r) oder mehrere(n) Verbindung(en) der Formel (IV) sowie eine(r) oder mehrere(n) Verbindung(en) der Formel (IV'),
dadurch gekennzeichnet, dass
- der Gesamtanteil der Verbindungen der Formel (II), (II'),(III), (IV) und/oder (IV') bzw. der Verbindungen der Formeln (IV) und (IV') größer als 40 Gew.-%, vorzugsweise größer als 55 Gew.-%, bevorzugt größer als 70 Gew.-%, weiter bevorzugt größer als 85 Gew.-%, besonders bevorzugt größer als 95 Gew.-%, bezogen auf die Gesamtmenge der in der Zusammensetzung enthaltenen 3,6,8,8-Tetramethyl-octahydro-3a,7-methano-azulen-5,6-diolketale ist,
und/oder dass
- das Gewichtsverhältnis der Verbindung(en) der Formel (IV) zu der/den Verbindung(en) der Formel (IV'), sofern vorhanden, im Bereich von 4 : 1 bis 1 : 10, vorzugsweise im Bereich von 2 : 1 bis 1 : 6, besonders bevorzugt im Bereich von 1 : 1 bis 1 : 3 liegt.

Für bevorzugt auszuwählende Verbindungen der Formel (I) (II), (II'), (III), (IV) und/oder (IV') gilt dabei das oben Gesagte entsprechend.

Die erfindungsgemäßen Verbindungen der Formel (I) zeichnen sich vorteilhafterweise durch ein hohes Aufziehvermögen (d.h. Eigenhaftung auf einem Substrat) und eine hohe Substantivität (d.h. Fähigkeit, aus einer, meist wässrigen, Phase heraus auf ein Substrat aufzuziehen bzw. auch nach einem Wasch- oder Spülvorgang auf einem Substrat zu verbleiben) aus. Dieser Effekt zeigt sich insbesondere auf Substraten wie Haut, Haar und textilen Fasern (z.B. Wolle, Baumwolle, Leinen, synthetische Fasern). Die Begriffe "Substantivität" und "Retention" werden z.B. in EP 1 201 738 A1 ausführlich beschrieben (vergleiche dort die Abschnitte [0004]-[0005]).

Diesen Erkenntnissen entsprechend betrifft ein weiterer Aspekt der vorliegenden Erfindung die Verwendung der erfindungsgemäßen Verbindungen bzw. einer erfindungsgemäßen Zusammensetzung (wie oben beschrieben) als Mittel zum Erhöhen der Substantivität und/oder Retention einer Riechstoffmischung (insbesondere gegenüber bzw. auf Haaren bzw. textilen Fasern), vorzugsweise einer Riechstoffmischung mit fruchtiger, blumiger, und/oder würziger Note.

Neben ihrem hohen Aufziehvermögen zeichnen sich die erfindungsgemäßen Verbindungen der Formel (I) auch durch ihre fixierenden Eigenschaften aus, d.h. sie fungieren vorteilhafterweise als Fixateur. Als Fixateur erhöhen die erfindungsgemäßen Verbindungen die Haftfestigkeit von anderen Riechstoffen, sei es durch deren Dampfdruckerniedrigung oder geruchliche Verstärkung (z.B. Absenkung des Schwellenwertes).

Ein weiterer Aspekt der vorliegenden Erfindung betrifft daher auch die Verwendung der erfindungsgemäßen Verbindungen (wie oben beschrieben) oder einer erfindungsgemäßen Zusammensetzung (wie oben beschrieben) als Fixateur.

Gegenstand der vorliegenden Erfindung ist somit auch die Verwendung einer erfindungsgemäßen Verbindung (wie oben beschrieben) oder einer erfindungsgemäßen Zusammensetzung (wie oben beschrieben) als
- Riechstoff bzw. Riechstoffmischung, vorzugsweise als Riechstoff bzw. Riechstoffmischung mit den Geruchsnoten Ambra und/oder Holz,
- Mittel zum Erhöhen der Substantivität und/oder der Retention einer Riechstoffmischung, oder
- Fixateur.
Gegenstand der vorliegenden Erfindung sind weiterhin Riechstoffmischungen, vorzugsweise Parfümöle, umfassend oder bestehend aus
- ein(em) oder mehrere(n) Verbindung(en) der Formel (II), (II'),(III) oder (IV), vorzugsweise der Formel (IV), oder eine(r) erfindungsgemäßen Zusammensetzung
   sowie
- einen/m oder mehrere(n) (weiteren) nicht der Formel (II), oder (IV), entsprechenden Riechstoff(en),
dadurch gekennzeichnet, dass
(i) die Menge der Verbindung(en) der Formel (II), oder (IV), vorzugsweise der Formel (IV), ausreicht, eine Ambranote und/oder Holznote, insbesondere eine weiße Ambranote ("white amber"), zu vermitteln,
und/oder dass
(ii) der/die (weiteren) nicht der Formel (II), oder (IV) entsprechenden Riechstoff(e) eine, mehrere oder sämtliche der Noten fruchtig, blumig, würzig, holzig, moschus und ambriert vermitteln und die Menge der Verbindung(en) der Formel (II), (II'),(III) oder (IV), vorzugsweise der Formel (IV), ausreicht, eine, mehrere oder sämtliche der Noten fruchtig, blumig, würzig, holzig, moschus und ambriert zu modifizieren und/oder zu verstärken,
und/oder dass
(iii) die Menge der Verbindung(en) der Formel (I) (II), (II'),(III) oder (IV), vorzugsweise der Formel (IV), ausreicht, der Riechstoffmischung einen Eindruck von Volumen, Komplexität, Eleganz und/oder Natürlichkeit zu verleihen, und/oder dass
(iv) die Menge der Verbindung(en) der Formel (II), (II'),(III) oder (IV), vorzugsweise der Formel (IV), ausreicht, um im Vergleich zu einer ansonsten identisch zusammengesetzten Riechstoffmischung ohne Verbindung(en) der Formel (II), (II'),(III) oder (IV) einen pflegenderen, harmonischeren, hochwertigeren und/oder natürlicheren Geruchseindruck zu bewirken.

Für bevorzugt auszuwählende Verbindungen der Formel (II), (II'),(III) oder (IV) gilt dabei das oben Gesagte entsprechend.

Eine bevorzugte Riechstoffmischung bzw. ein bevorzugtes Parfümöl ist dadurch gekennzeichnet, dass die Menge der Verbindung(en) der Formel (II), (II'),(III) oder (IV), vorzugsweise der Formel (IV), im Bereich von 0,0001 bis 25 Gew.-%, vorzugsweise im Bereich von 0,001 bis 15 Gew.-%, bezogen auf das Gesamtgewicht der Riechstoffmischung liegt.

Erfindungsgemäße Riechstoffmischungen, insbesondere Parfümöle, umfassen vorzugsweise zwei, drei, vier, fünf, sechs, sieben, acht, neun, zehn oder mehr Riechstoffe, bevorzugt ausgewählt aus den nachfolgend genannten Stoffen:
Riechstoffe, die in Steffen Arctander, Perfume and Flavor Chemicals, Eigenverlag, Montclair, N. J. 1969; H. Surburg, J. Panten, Common Fragrance and Flavor Materials, 5th Edition, Wiley-VCH, Weinheim 2006 genannt sind.

Als mit den erfindungsgemäßen Verbindungen und Zusammensetzungen bevorzugt kombinierbare Riechstoffe bzw. Riechstoff enthaltende Substanzen seien insbesondere genannt:
Extrakte aus natürlichen Rohstoffen wie Etherische Öle, Concretes, Absolues, Resine, Resinoide, Balsame, Tinkturen wie z. B. Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-Absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-Citriodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl, Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepresst; Linaloeöl; Litsea-Cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnussöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-Tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl; sowie Fraktionen davon bzw. daraus isolierte Inhaltsstoffe;
einzelne Riechstoffe aus der Gruppe der Kohlenwasserstoffe, wie z. B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (*E,Z*)-1,3,5-Undecatrien;
aus der Gruppe der aliphatischen Alkohole, wie z. B. Hexanol; Octanol; 3-Octanol; 2,6-Dimethylheptanol; 2-Methylheptanol, 2-Methyloctanol; (*E*)-2-Hexenol; (*E*)- und (*Z*)-3-Hexenol; 1-Octen-3-ol; Gemisch von 3,4,5,6,6-Pentamethyl-3/4-hepten-2-ol und 3;5,6,6-Tetramethyl-4-methyleneheptan-2-ol; (*E,Z*)-2,6-Nonadienol; 3,7-Dimethyl-7-methoxyoctan-2-ol; 9-Decenol; 10-Undecenol; 4-Methyl-3-decen-5-ol;
aus der Gruppe der aliphatischen Aldehyde und deren Acetale wie z. B. Hexanal; Heptanal; Octanal; Nonanal; Decanal; Undecanal; Dodecanal; Tridecanal; 2-Methyloctanal; 2-Methylnonanal; (*E*)-2-Hexenal; (*Z*)-4-Heptenal; 2,6-Dimethyl-5-heptenal; 10-Undecenal; (*E*)-4-Decenal; 2-Dodecenal; 2,6,10-Trimethyl-5,9-undecadienal; Heptanal-diethylacetal; 1,1-Dimethoxy-2,2,5-trimethyl-4-hexen; Citronellyloxyacetaldehyd;
aus der Gruppe der aliphatischen Ketone und deren Oxime wie z. B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on;
aus der Gruppe der aliphatischen schwefelhaltigen Verbindungen wie z. B. 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
aus der Gruppe der aliphatischen Nitrile wie z. B. 2-Nonensäurenitril; 2-Tridecensäurenitril; 2,12-Tridecensäurenitril; 3,7-Dimethyl-2,6-octadien-säurenitril; 3,7-Dimethyl-6-octensäurenitril;
aus der Gruppe der aliphatischen Carbonsäuren und deren Ester wie z. B. (*E*)- und (*Z*)-3-Hexenylformiat; Ethylacetoacetat; Isoamylacetat; Hexylacetat; 3,5,5-Trimethylhexylacetat; 3-Methyl-2-butenylacetat; (*E*)-2-Hexenylacetat; (*E*)- und (*Z*)-3-Hexenylacetat; Octylacetat; 3-Octylacetat; 1-Octen-3-ylacetat; Ethylbutyrat; Butylbutyrat; Isoamylbutyrat; Hexylbutyrat; (*E*)- und (*Z*)-3-Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(*E*,*Z*)-2,4-decadienoat; Methyl-2-octinat; Methyl-2-noninat; Allyl-2-isoamyloxyacetat; Methyl-3,7-dimethyl-2,6-octadienoat;
aus der Gruppe der acyclischen Terpenalkohole wie z. B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol; 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate, 3-Methyl-2-butenoate;
aus der Gruppe der acyclischen Terpenaldehyde und -ketone wie z. B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
aus der Gruppe der cyclischen Terpenalkohole wie z. B. Menthol; Isopulegol, α-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate, 3-Methyl-2-butenoate;
aus der Gruppe der cyclischen Terpenaldehyde und -ketone wie z. B. Menthon; Isomenthon; 8-Mercaptomenthan-3-on; Carvon; Campher; Fenchon; α-lonon; β-Ionon; α-n-Methylionon; β-n-Methylionon; α-Isomethylionon; β-Isomethylionon; α-Iron; α-Damascon; β-Damascon; β-Damascenon; γ-Damascon; δ-Damascon; 1-(2,4,4-Trimethyl-2-cyclohexen-1-yl)-2-buten-1-on; 1,3,4,6,7,8a-Hexahydro-1,1,5,5-tetramethyl-2*H*-2,4a-methanonaphthalen-8(5*H*)-on; Nootkaton; Dihydronootkaton; α-Sinensal; β-Sinensal; acetyliertes Cedernholzöl (Methylcedrylketon);
aus der Gruppe der cyclischen Alkohole wie z. B. 4-*tert*-Butylcyclohexanol; 3,3,5-Trimethylcyclohexanol; 3-lsocamphylcyclohexanol; 2,6,9-Trimethyl-(*Z*2,*Z*5,*E*9)-cyclododecatrien-1-ol; 2-lsobutyl-4-methyltetrahydro-2H-pyran-4-ol;
aus der Gruppe der cycloaliphatischen Alkohole wie z. B. α,3,3-Trimethyl-cyclohexylmethanol; 2-Methyl-4-(2,2,3-trimethyl-3-cyclopent-1-yl)butanol; 2-Methyl-4-(2,2,3-trimethyl⁻3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
aus der Gruppe der cyclischen und cycloaliphatischen Ether wie z. B. Cineol; Cedrylmethylether; Cyclododecylmethylether; (Ethoxymethoxy)cyclododecan; α-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
aus der Gruppe der cyclischen Ketone wie z. B. 4-*tert*-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-*tert-*Pentylcyclohexanon; 5-Cyclohexadecen-1-on; 6,7-Dihydro-1,1,2,3,3-pentamethyl-4(5*H*)-indanon; 9-Cycloheptadecen-1-on; Cyclopentadecanon; Cyclohexadecanon;
aus der Gruppe der cycloaliphatischen Aldehyde wie z. B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
aus der Gruppe der cycloaliphatischen Ketone wie z. B. 1-(3,3-Dimethylcyclohexyl)-4-penten-1-on; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; *tert*-Butyl-(2,4-dimethyl-3-cyclohexen-1-yl)keton;
aus der Gruppe der Ester cyclischer Alkohole wie z.B. 2-*tert-*Butylcyclohexylacetat; 4-*tert*-Butylcyclohexylacetat; 2-*tert*-Pentylcyclohexyl-acetat; 4-*tert*-Pentylcyclohexylacetat; Decahydro-2-naphthylacetat; 3-Pentyl-tetrahydro-2*H-*pyran-4-ylacetat; Decahydro-2,5,5,8a-tetramethyl-2-naphthyl-acetat; 4,7-Methano-3a,4,5,6,7,7a-hexahydro-5- bzw. -6-indenylacetat; 4,7-Methano-3a,4,5,6,7,7a-hexahydro-5- bzw. -6-indenylpropionat; 4,7-Methano-3a,4,5,6,7,7a-hexahydro-5- bzw. -6-indenylisobutyrat; 4,7-Methanooctahydro-5- bzw. -6-indenylacetat;
aus der Gruppe der Ester cycloaliphatischer Carbonsäuren wie z. B. Allyl-3-cyclohexylpropionat; Allylcyclohexyloxyacetat; Methyldihydrojasmonat; Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexen-carboxylat; Ethyl-2-methyl-1,3-Dioxolan-2-acetat;
aus der Gruppe der aromatischen Kohlenwasserstoffe wie z. B. Styrol und Diphenylmethan;
aus der Gruppe der araliphatischen Alkohole wie z. B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
aus der Gruppe der Ester von araliphatischen Alkoholen und aliphatischen Carbonsäuren wie z. B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; α-Trichlormethylbenzylacetat; α,α-Dimethylphenylethylacetat; α,α-Dimethylphenylethylbutyrat; Cinnamylacetat; 2-Phenoxyethylisobutyrat; 4-Methoxybenzylacetat;
aus der Gruppe der araliphatischen Ether wie z. B. 2-Phenylethylmethylether; 2-Phenylethylisoamylether; 2-Phenylethyl-1-ethoxyethylether; Phenylacetaldehyd-dimethylacetal; Phenylacetaldehyd-diethylacetal; Hydratropaaldehyddimethylacetal; Phenylacetaldehyd-glycerinacetal; 2,4,6-Trimethyl-4-phenyl-1,3-dioxane; 4,4a,5,9b-Tetrahydroindeno[1,2-d]-m-dioxin; 4,4a,5,9b-Tetrahydro-2,4-dimethylindeno[1,2-d]-m-dioxin;
aus der Gruppe der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-*tert-*butylphenyl)propanal; 3-(4-*tert*-Butylphenyl)propanal; Zimtaldehyd; α-Butylzimtaldehyd; α-Amylzimtaldehyd; α-Hexylzimtaldehyd; 3-Methyl-5-phenyl-pentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
aus der Gruppe der aromatischen und araliphatischen Ketone wie z. B. Acetophenon; 4-Methylacetophenon; 4-Methoxyacetophenon; 4-*tert*-Butyl-2,6-dimethylacetophenon; 4-Phenyl-2-butanon; 4-(4-Hydroxyphenyl)-2-butanon; 1-(2-Naphthalenyl)ethanon; Benzophenon; 1,1,2,3,3,6-Hexamethyl-5-indanyl-methylketon; 6-*tert*-Butyl-1,1-dimethyl-4-indanylmethylketon; 1-[2,3-dihydro-1,1,2,6-tetramethyl-3-(1-methylethyl)-1*H-*5-indenyl]ethanon; 5',6',7',8'-Tetrahydro-3',5',5',6',8',8'-hexamethyl-2-acetonaphthon;
aus der Gruppe der aromatischen und araliphatischen Carbonsäuren und deren Ester wie z. B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethyl-phenylacetat; Methylcinnamat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; *cis*-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
aus der Gruppe der stickstoffhaltigen aromatischen Verbindungen wie z. B. 2,4,6-Trinitro-1,3-dimethyl-5-*tert*-butylbenzol; 3,5-Dinitro-2,6-dimethyl-4-*tert-*butylacetophenon; Zimtsäurenitril; 5-Phenyl-3-methyl-2-pentensäurenitril; 5-Phenyl-3-methylpentansäurenitril; Methylanthranilat; Methy-*N*-methyl-anthranilat; Schiff'sche Basen von Methylanthranilat mit 7-Hydroxy-3,7-dimethyloctanal, 2-Methyl-3-(4-*tert*-butylphenyl)propanal oder 2,4-Dimethyl-3-cyclohexencarbaldehyd; 6-lsopropylchinolin; 6-lsobutylchinolin; *6-sec-*Butylchinolin; Indol; Skatol; 2-Methoxy-3-isopropylpyrazin; 2-Isobutyl-3-methoxypyrazin; 4-(4,8-Dimethyl-3,7-nonadienyl)-pyridin;
aus der Gruppe der Phenole, Phenylether und Phenylester wie z. B. Estragol; Anethol; Eugenol; Eugenylmethylether; Isoeugenol; Isoeugenylmethylether; Thymol; Carvacrol; Diphenylether; β-Naphthylmethylether; β-Naphthylethylether; β-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
aus der Gruppe der heterocyclischen Verbindungen wie z. B. 2,5-Dimethyl-4-hydroxy-2*H*-furan-3-on; 2-Ethyl-4-hydroxy-5-methyl-2*H*-furan-3-on; 3-Hydroxy-2-methyl-4*H*-pyran-4-on; 2-Ethyl-3-hydroxy-4*H*-pyran-4-on;
aus der Gruppe der Lactone wie z. B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 1,15-Pentadecanolid; *cis-* und *trans*-11-Pentadecen-1,15-olid; *cis-* und *trans*-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Die erfindungsgemäßen Verbindungen oder eine erfindungsgemäße Zusammensetzung (wie oben beschrieben) beeinflussen die sensorischen Eigenschaften von Riechstoffmischungen auf diverse Art und Weise (siehe dazu auch die untenstehenden Vergleichsbeispiele).

Eine bevorzugte erfindungsgemäße Riechstoffmischung bzw. ein bevorzugtes erfindungsgemäßes Parfümöl ist dadurch gekennzeichnet, dass die Riechstoffmischung bzw. das Parfümöl
(i) (zudem) einen oder mehrere nicht der Formel (II), (II'),(III) oder (IV) entsprechende(n) Holz- und/oder Ambra-Riechstoff(e) und/oder
(ii) einen oder mehrere Moschusriechstoff(e) enthält.

Der oder die Moschusriechstoffe des Bestandteils (ii) sind Riechstoffe, die einen Moschusgeruch aufweisen. Solche Riechstoffe sind dem Fachmann bekannt, da "Moschus" (musk) eine wichtige Geruchsrichtung in der Parfümerie darstellt.

Es ist erfindungsgemäß bevorzugt, dass der/die Moschusriechstoff(e) ausgewählt ist/sind aus der Gruppe bestehend aus polycyclischen und macrocyclischen Moschusriechstoffen, vorzugsweise aus der Gruppe bestehend aus macrocyclischen C₁₄-C₁₈-Ketonen und macrocyclischen C₁₄-C₁₈-Lactonen.

Im Rahmen der vorliegenden Erfindung vorteilhaft eingesetzte Moschusriechstoffe sind in Tabelle 1 aufgeführt.

**Tabelle 1:**

| TYP | Produkt / Markenname | Name / CAS-Name |
|---|---|---|
| MACRO | EXALTENON | 4-Cyclopentadecen-1-one (4Z)- ; 4-Cyclopentadecen-1-on |
| MACRO | CIVETON | 9-Cycloheptadecen-1-on, (9Z)- |
| MACRO | CYCLOHEXADECANOLID, DIHYDROAMBRETTOLID | Oxacycloheptadecan-2-on, ω-Hexadecanolid |
| MACRO | ETHYLENDODECANDIOAT | 1,4-Dioxacyclohexadecane-5,16-dion |
| MACRO | GLOBALIDE® | Oxacyclohexadecen-2-on; 15-Pentadec-(11/12)-enolid |
| MACRO | ETHYLENBRASSYLAT | 1,4-Dioxacycloheptadecane-5,17-dion |
| MACRO | MUSCON | 3-Methy-cyclopentadecanon |
| MACRO | AMBRETTOLID | Oxacycloheptadec-10-en-2-on |
| MACRO | MUSCENON | 3-Methyl-cyclopentadecenon |
| MACRO | VELVIONE®, AMBRETONE | 5-Cyclohexadecen-1-on |
| MACRO | AURELIONE® | 7/8-Cyclohexadecen-1-on |
| MACRO | GLOBANONE® | 8-Cyclohexadecen-1-on |
| MACRO | ISOMUSCONE® | Cyclohexadecanon |
| MACRO | EXALTOLID, MACROLIDE® | Oxacyclohexadecan-2-on |
| MACRO | COSMONE® | 3-Methyl-(5E/Z)-cyclotetradecen-1-on |
| POLY | TRASEOLIDE® | 1-[2,3-Dihydro-1,1,2,6-tetramethyl-3-(1-methylethyl)-1 H-inden-5- yl]-ethanon |
| POLY | PHANTOLIDE® | 1-(2,3-Dihydro-1,1,2,3,3,6-hexamethyl-1H-inden-5-yl)-ethanon |
| POLY | TONALIDE® | 1-(5,6,7,8-Tetrahydro-3,5,5,6,8,8-hexamethyl-2-naphthalenyl)-ethanon |
| POLY | CRYSOLIDE | 1-[6-(1,1-Dimethylethyl)-2,3-dihydro-1,1-dimethyl-1H-inden-4-yl]-ethanon |
| POLY | CHROMANOLIDE® | Tetradecansäure, 1-methylethyl ester; Cyclopenta[g]-2-benzopyran, 1,3,4,6,7,8-hexahydro-4,6,6,7,8,8-hexamethyl- |
| POLY | GALAXOLIDE® | Cyclopenta[g]-2-benzopyran, 1,3,4,6,7,8-hexahydro-4,6,6,7,8,8-hexamethyl- |
| ALICYC | HELVETOLIDE® | 1-Propanol, 2-[1-(3,3-dimethylcyclohexyl)ethoxy]-2-methyl-, 1-propanoat |
| NITRO | MOSKENE | 2,3-Dihydro-1,1,3,3,5-pentamethyl-4,6-dinitro-1H-Inden |
| NITRO | MUSK TIBETENE | 1-(1,1-Dimethylethyl)-3,4,5-trimethyl-2,6-dinitrobenzol |
| NITRO | ORINOX | 1-[4-(1,1-Dimethylethyl)-2,6-dimethylphenyl]-ethanon |
| NITRO | MUSK XYLOL | 1-(1,1-Dimethylethyl)-3,5-dimethyl-2,4,6-trinitrobenzol |
| NITRO | MUSK KETONE | 1-[4-(1,1-Dimethylethyl)-2,6-dimethyl-3,5-dinitrophenyl]-ethanon |
| NITRO | MUSK ALPHA | 1,3-Dibromo-2-methoxy-4-methyl-5-nitrobenzol |

| | | |
|---|---|---|
| MACRO = macrocyclische Moschusriechstoffe NITRO = Nitro-Mochusriechstoffe POLY = polycyclische Mochusriechstoffe ALICYC = alicyclischer Mochusriechstoff | | |

Weiterhin ist es erfindungsgemäß bevorzugt, dass das Gewichtsverhältnis an Moschusriechstoffen zu der/den Verbindung(en) der Formel (II), oder (IV), vorzugsweise der Formel (IV), gleich oder größer als 10 : 1 ist, vorzugsweise im Bereich von 10 : 1 bis 100.000 : 1, weiter bevorzugt im Bereich von 50 : 1 bis 100.000 : 1, besonders bevorzugt im Bereich von 100 : 1 bis 100.000 : 1 liegt.

Besonders bevorzugt sind erfindungsgemäße Riechstoffmischungen, die 3-Methylcyclopentadecenon (Muscenon), 15-Pentadec-(11/12)-enolid (Globalide)®, Ethylenbrassylat, Oxacyclohexadecan-2-on (Macrolide®), Cyclohexadecanon (Isomuscone®), 8-Cyclohexadecanon (Globanone®), (7/8)- Cyclohexadecanon (Aurelione®) und/oder Mischungen davon enthalten.

Wie bereits erwähnt, eignen sich die erfindungsgemäßen Verbindungen wegen ihrer olfaktorischen Eigenschaften besonders gut für den Einsatz in Riechstoffmischungen und insbesondere Parfümölen. Die Verbindungen können dabei alleine oder in Mischungen in entsprechenden Riechstoffmischungen mit weiteren Einzelriechstoffen oder aber einer Vielzahl weiterer Riechstoffe zusammen eingesetzt werden. Besonders vorteilhaft lassen sich die erfindungsgemäßen Verbindungen mit anderen Riechstoffen, vorzugsweise ausgewählt aus den bereits oben bzw. weiter unten genannten Riechstoffen, in unterschiedlichen Mengenverhältnissen zu neuartigen Riechstoffmischungen bzw. Parfümen kombinieren.

Ein weiterer Aspekt der vorliegenden Erfindung betrifft daher ein Verfahren zum Verstärken eines Geruchs oder mehrerer Gerüche ausgewählt aus der Gruppe bestehend aus fruchtig, blumig, würzig, holzig, moschus und ambriert, umfassend den folgenden Schritt:
(i) Vermischen von einem oder mehreren Riechstoff/en mit einer, mehreren oder sämtlichen der Noten ausgewählt aus der Gruppe bestehend aus fruchtig, blumig, würzig, holzig, moschus und ambriert mit einer oder mehreren Verbindung(en) der Formel (II), (II'),(III) oder (IV), vorzugsweise der Formel (IV), einer erfindungsgemäßen Zusammensetzung oder Riechstoffmischung, vorzugsweise einem Parfümöl, in einer Menge, die ausreicht, den bzw. die Geruchseindruck bzw. Geruchseindrücke der/des Riechstoffe(s) zu verstärken.

Die erfindungsgemäßen Verbindungen, erfindungsgemäße Zusammensetzungen bzw. erfindungsgemäße Riechstoffmischungen werden vorzugsweise zur Herstellung parfümierter Produkte (parfümierte Artikel) eingesetzt. Die sensorischen Eigenschaften ebenso wie die stofflichen Eigenschaften (wie Löslichkeit in gängigen Lösungsmitteln und Kompatibilität mit gängigen weiteren Bestandteilen derartiger Produkte) sowie die toxikologische Unbedenklichkeit der erfindungsgemäßen Verbindung unterstreichen ihre besondere Eignung für die genannten Einsatzzwecke.

Gegenstand der vorliegenden Erfindung sind somit auch parfümierte Produkte enthaltend eine oder mehrere erfindungsgemäße Verbindung(en) (II), (II'),(III) oder (IV), insbesondere eine oder mehrere Verbindung(en) der Formel (IV), eine erfindungsgemäße Zusammensetzung oder Riechstoffmischung, vorzugsweise in einer sensorisch wirksamen Menge.

Bevorzugte erfindungsgemäße parfümierte Produkte sind ausgewählt aus der Gruppe bestehend aus:
Parfüm-Extraits, Eau de Parfums, Eau de Toilettes, Rasierwässer, Eau de Colognes, Pre-shave-Produkte, Splash-Colognes, parfümierte Erfrischungstücher, Parfüms für saure, alkalische und neutrale Reinigungsmittel, Waschmittel, Waschtabletten, Desinfektionsmitteln, sowie von Luftverbesserern, Aerosolsprays, Wachse und Polituren, sowie Körperpflegemitteln, Badeölen, kosmetischen Emulsionen, wie z.B. Hautcremes- und -lotionen, Sonnenschutzcremes- und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, festigende Haarlotionen, Haarspülungen, Haarfärbemitteln, Haarverformungsmitteln und Haarglättungsmitteln, Haarwässern, Haarcremes und -lotionen, Deodorantien und Antiperspirantiens, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repel-lentien, Treibstoffen.

Erfindungsgemäße Riechstoffmischungen enthaltend die erfindungsgemäßen Verbindungen oder eine wie oben definierte erfindungsgemäße Zusammensetzung können allgemein (z.B. in konzentrierter Form, in Lösungen oder in unten beschriebener modifizierter Form) verwendet werden für die Herstellung von z.B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-ÖI- und vom Wasser-in-Öl-in-Wasser-Typ wie z.B. Hautcremes- und -lotionen, Gesichtscremes und -lotionen, Sonnenschutzcremes und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, festigen Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien, Treibstoffen.

Die erfindungsgemäßen zuvor genannten Riechstoffmischungen bzw. die erfindungsgemäß in den entsprechenden Produkten einzusetzenden Riechstoffmischungen können in flüssiger Form, unverdünnt oder mit einem Lösungsmittel verdünnt für Parfümierungen eingesetzt werden. Geeignete Lösungsmittel hierfür sind z.B. Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat usw. Für die genannten Lösungsmittel gilt, dass diese im Rahmen des vorliegenden Textes im Falle des Vorhandenseins eigener olfaktorischer Eigenschaften ausschließlich dem Bestandteil "Lösungsmittel" und nicht den "Riechstoffen" zuzuordnen sind.

Die in den erfindungsgemäßen parfümierten Produkten enthaltenen erfindungsgemäßen Verbindungen, eine wie oben beschriebene erfindungsgemäße Zusammensetzung oder eine wie oben beschriebene erfindungsgemäße Riechstoffmischung können dabei in einer bevorzugten Ausführungsform an einem Trägerstoff absorbiert sein, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer und Cellulose-basierende Stoffe sein.

Die in den erfindungsgemäßen parfümierten Produkten enthaltenen erfindungsgemäßen Verbindungen, eine wie oben beschriebene erfindungsgemäße Zusammensetzung oder eine wie oben definierte erfindungsgemäße Riechstoffmischung können auch mikroverkapselt, sprühgetrocknet, als Einschluss-Komplexe oder als Extrusions-Produkte vorliegen und in dieser Form dem zu parfümierenden Produkt, bzw. Artikel, hinzugefügt werden.

Gegebenenfalls können die Eigenschaften der derart modifizierten Riechstoffmischungen durch sogenanntes "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden.

Die Mikroverkapselung der Riechstoffmischungen kann beispielsweise durch das sogenannte Koazervationsverfahren mit Hilfe von Kapselmaterialien, z.B. aus polyurethan-artigen Stoffen oder Weichgelatine, erfolgen. Die sprühgetrockneten Parfümöle können beispielsweise durch Sprühtrocknung einer das Parfümöl enthaltenden Emulsion bzw. Dispersion hergestellt werden, wobei als Trägerstoffe modifizierte Stärken, Proteine, Dextrin und pflanzliche Gummen verwendet werden können. Einschluss-Komplexe können z.B. durch Eintragen von Dispersionen von der Riechstoffmischung und Cyclodextrinen oder Harnstoffderivaten in ein geeignetes Lösungsmittel, z.B. Wasser, hergestellt werden. Extrusions-Produkte können durch Verschmelzen der Riechstoffmischung mit einem geeigneten wachsartigen Stoff und durch Extrusion mit nach-folgender Erstarrung, ggf. in einem geeigneten Lösungsmittel, z.B. Isopropanol, erfolgen.

Die erfindungsgemäßen Riechstoffmischungen können demnach, wie bereits erwähnt, in konzentrierter Form, in Lösungen oder in oben beschriebener modifizierter Form für die Herstellung der entsprechenden erfindungsgemäßen parfümierten Artikel verwendet werden

Zutaten, mit denen die erfindungsgemäßen Verbindungen, eine wie oben beschriebene erfindungsgemäße Zusammensetzung oder eine wie oben beschriebene erfindungsgemäße Riechstoffmischung, vorzugsweise kombiniert werden können, sind beispielsweise: Konservierungsmittel, Abrasiva, Antiakne-Mittel, Mittel gegen Hautalterung, antibakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, schweißhemmende Mittel, antiseptische Mittel, Antistatika, Binder, Puffer, Trägermaterialien, Chelatbildnder, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Filmbildner, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Silicone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UV-absorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, α-Hydroxysäuren, Polyhydroxyfettsäuren, Verflüssiger, Farbstoffe, farbschützende Mittel, Pigmente, Antikorrosiva, Aromen, Geschmackstoffe, Polyole, Tenside, Elektrolyte, organische Lösungsmittel oder Silikonderivate.

Im Folgenden werden weitere erfindungsgemäße Aspekte im Zusammenhang mit erfindungsgemäßen Verbindungen, einer erfindungsgemäßen Zusammensetzung (wie oben beschrieben) bzw. einer erfindungsgemäßen Riechstoffmischung (wie oben beschriebene) erläutert.

Wie oben bereits ausgeführt, ist für tensidhaltige parfümierte Produkte die Substantivität eines Riechstoffs bzw. einer Riechstoffmischung gegenüber den bzw. deren Retention am Substrat, insbesondere Haaren oder textilen Fasern, eine weitere wichtige anwendungstechnische Anforderung.

Durch Zusatz der erfindungsgemäßen Verbindungen bzw. einer erfindungsgemäßen Zusammensetzung (wie oben beschrieben) zu einer vorgegebenen Riechstoffmischung nur geringer Substantivität und/oder Retention werden diese Eigenschaften in besonders vorteilhafter Weise verbessert. So lässt sich beispielsweise eine zwar fruchtig, blumig, und/oder würzig duftende, aber aufgrund nur mangelhafter Substantivität der enthaltenen Duftstoffe nicht zum Weitergeben eines fruchtigen, blumigen und/oder würzigen Geruchs an Wäsche (textile Fasern) oder Haare geeignete wässrige Waschlösung (bzw. ein entsprechendes Waschmittel oder Shampoo oder dergleichen) durch Zusatz der erfindungsgemäßen Verbindungen bzw. einer erfindungsgemäßen Zusammensetzung (wie oben beschrieben) in eine Lösung überführen, die einen fruchtigen, blumigen und/oder würzigen Geruch hervorragend weitergibt. An den behandelten Substraten (Haare bzw. textile Fasern) haftet der fruchtige, blumige und/oder würzige Geruch lange an.

Die erfindungsgemäßen Verbindungen bzw. eine erfindungsgemäße Zusammensetzung (wie oben beschrieben) enthaltende Riechstoffmischungen (wie oben beschrieben) zeichnen sich durch eine hohe Substantivität aus. Dieser Effekt zeigt sich insbesondere auf Substraten wie Haut, Haar und textilen Fasern (z.B. Wolle, Baumwolle, Leinen, synthetische Fasern).

Dieser Effekt wird weiter unten im Rahmen der anwendungstechnischen Beispiele näher erläutert.

Gegenstand der vorliegenden Erfindung ist somit auch ein parfümiertes Produkt (wie oben beschrieben), welches ein oder mehrere Tenside enthält.

Beispielsweise ist ein erfindungsgemäßes parfümiertes Produkt ein schwach saures, alkalisches oder neutrales Reinigungsmittel, das insbesondere aus der Gruppe ausgewählt ist bestehend aus
- Allzweckreinigern, Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln,
- Luftverbesserern in flüssiger, gelartiger oder auf einem festen Träger aufgebrachter Form oder als Aerosolspray,
- Wachsen oder eine Polituren, die insbesondere aus der Gruppe ausgewählt ist bestehend aus Möbelpolituren, Fußbodenwachsen und Schuhcremes
und
- Körperpflegemitteln, das insbesondere aus der Gruppe ausgewählt ist bestehend aus Duschgelen und Shampoos.

Die beschriebenen positiven Eigenschaften tragen dazu bei, dass die erfindungsgemäßen Verbindungen, eine erfindungsgemäße Mischung (wie oben definiert) bzw. eine erfindungsgemäße Riechstoffmischung (wie oben definiert) besonders bevorzugt in Wasch- und Reinigungsmitteln, Hygiene- oder Pflegeprodukten, insbesondere im Bereich der Körper- und Haarpflege, der Kosmetik und des Haushalts eingesetzt werden.

Besonders bevorzugte erfindungsgemäße parfümierte Produkte sind daher Wasch- und Reinigungsmittel, Hygiene- oder Pflegeprodukte, insbesondere im Bereich der Körper- und Haarpflege, der Kosmetik und des Haushalts.

Eine erfindungsgemäße Riechstoffmischung wird erfindungsgemäß vorzugsweise hergestellt, indem die erfindungsgemäßen Verbindung oder eine wie oben definierte erfindungsgemäße Mischung mit dem oder den weiteren Riechstoffen sowie gegebenenfalls weiteren Bestandteilen der Riechstoffmischung vermischt werden.

Gemäß einer bevorzugten Ausführungsform wird eine erfindungsgemäße Riechstoffmischung wie oben beschrieben hergestellt, wobei die erfindungsgemäßen Verbindungen in einer Menge eingesetzt wird, die ausreicht, um in der Riechstoffmischung eine oder vorzugsweise beide der Geruchsnoten Ambra und Holz zu vermitteln, zu modifizieren und/oder zu verstärken.

Wie bereits erwähnt, können die erfindungsgemäßen Verbindungen als Mittel zum Versehen von Haaren und/oder textilen Fasern einer oder beider Geruchsnoten Ambra und/oder Holz verwendet werden.

Dementsprechend betrifft ein weiterer Aspekt der vorliegenden Erfindung ein Verfahren zum Versehen von Haaren oder textilen Fasern mit einer, mehrerer oder sämtlichen der Noten ausgewählt aus der Gruppe bestehend aus fruchtig, blumig, würzig, holzig, moschus und ambriert, umfassend die folgenden Schritte:
(i) Bereitstellen einer erfindungsgemäßen Riechstoffmischung, wobei der/die (weiteren) nicht der Formel (II), (II'),(III) oder (IV), entsprechenden Riechstoff(e) eine, mehrere oder sämtliche der Noten fruchtig, blumig, würzig, holzig, moschus und ambriert vermitteln und die Menge der Verbindung(en) der Formel (II), (II'),(III) oder (IV), vorzugsweise der Formel (IV), ausreicht, um eine, mehrere oder sämtliche der Noten fruchtig, blumig, würzig, holzig, moschus und ambriert zu modifizieren und/oder zu verstärken, und
(ii) Applizieren der Riechstoffmischung auf das Haar oder die textilen Fasern.

Für bevorzugte Bestandteile einer hierbei zu verwendenden Riechstoffmischung gilt dabei das oben Gesagte entsprechend.

Die vorliegende Erfindung soll im Folgenden anhand ausgewählter Beispiele näher erläutert werden.

### Beispiele:

Sofern nicht anders angegeben beziehen sich Anteile und Prozente auf das jeweilige Gewicht.

### Verwendete Abkürzungen:

Dipropylenglycol (DPG), Diethylphthalat (DEP), Triethylcitrat (TEC).

Für Erläuterungen der Produktnamen der Riechstoffe siehe z.B. S. Arctander, Perfume and Flavor Materials, Vol. I und II, Montclair, N. J., 1969, Selbstverlag oder H. Surburg, J. Panten, "Common Fragrance and Flavor Materials", 5th. Ed., Wiley-VCH, Weinheim 2006.

Verwendet wurde *cis*-Cedrandiol, das durch Epoxidation und anschließende Ringöffnung aus (-)-α-Cedren erhalten wurde. Laut NMR-Untersuchung liegt folgende Struktur für das Diol vor:

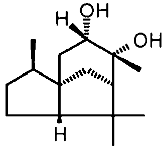

### Beispiel 1

### Umsetzung von cis-Cedrandiol mit 1-Methoxy-propan-2-on

24 g (0,1 Mol) cis-Cedrandiol (eingesetzt wurden 26 g mit einem GC-Gehalt von 92%), 127,6 g (1,45 mol) 1-Methoxy-propan-2-on, 31,8 g (0,3 mol) Orthoameisensäuretrimethylester und 0,4 g konzentrierte Schwefelsäure wurden 24 Stunden bei 10°C gerührt. Nach dem Reaktionsende wurde Methyl-tert.-butylether zugegeben, mit einer 5%-igen Natriumhydrogencarbonat-Lösung gewaschen und die Leichtsieder abdestilliert. Nach Hinzufügen von n-Hexan kristallisierte nicht umgesetztes cis-Cedrandiol aus, das durch Filtration abgetrennt wurde. Nach Einengen des Filtrats wurden 30,4 g Rohprodukt erhalten. Gemäß GC-Analyse lag der Gehalt der Verbindungen der Formeln (VIII) und (IX) in dem erhaltenen Produkt bei insgesamt 51% (entsprechend 50% Ausbeute der Theorie). Durch Lösen des Rohproduktes in Dipropylenglykol (DPG) wurde hieraus eine Lösung mit einem Gehalt von 10 Gew.-% an Verbindungen der Formel (VIII) und (IX) (mit einem Verhältnis von (VIII) : (IX) = 1 : 2,3) hergestellt.

Für analytische Untersuchungen wurden aus dem Rohprodukt die stereoisomeren Verbindungen der Formel (VIII) und (IX) mittels Flüssigkeitschromatographie an Kieselgel 60 (0,04-0,063 mm) mit dem Elutionsmittel Cyclohexan/Essigsäureethylester (60/1) voneinander getrennt. Die Verbindung der Formel (VIII) wurde mit einer Reinheit von 99,4% und die Verbindung der Formel (IX) mit einer Reinheit von 96,9% erhalten.

### Verbindung der Formel (VIII):

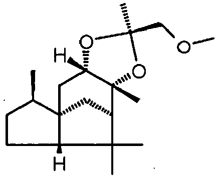

¹H-NMR (400 MHz, C₆D₆): 3.95 (dd, J=8.5, 7.2 Hz,1H), 3.58 (dq, J= 10.3, 0.5 Hz, 1 H), 3.51 (dq, *J =* 10.3, 0.5 Hz, 1 H), 3.28 (s, 3H), 2.23 (dt, *J =* 12.1, 1.0 Hz, 1 H), 1.98 (d, *J* = 4.5 Hz, 1 H), 1.86-1.76 (m, 2H), 1.754 (q, *J* = 0.3 Hz, 3H), 1.75-1.69 (m, 1 H), 1.62 (h, *J =* 6.9 Hz, 1 H), 1.49 (s, 3H), 1.44 (ddd, *J* = 12.3, 4.8, 2.0 Hz, 1 H), 1.42-1.35 (m,1 H), 1.36-1.25 (m, 2H), 1.23-1.11 (m, 1 H), 0.96 (s, 3H), 0.89 (s, 3H), 0.76 (d, J= 7.1 Hz, 3H) ppm.
¹³C-NMR (C₆D₆): 109.86, 85.13, 80.02, 78.58, 59.15, 58.70, 57.83, 52.53, 42.36, 42.23, 41.65, 38.91, 36.17, 31.17, 28.79, 28.19, 25.61, 25.19, 15.59 ppm.

Geruch: sehr stark Ambra, Holz, erinnert an weiße Ambra, sehr lange Haftung Verbindung der Formel (IX):

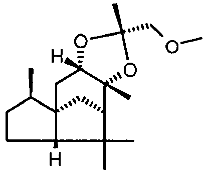

¹H-NMR (400 MHz, C₆D₆): 3.99 (dd, *J* = 8.9, 6.8 Hz,1H), 3.60 (d, *J* = 10.2 Hz, 1 H), 3.57 (d, *J* = 10.2 Hz, 1 H), 3.26 (d, *J* = 0.5, 3H), 2.23 (d, *J* = 11.9 Hz, 1 H), 1.99 (d, *J* = 4.5 Hz, 1 H), 1.81 (ddd, *J* = 13.5, 8.9, 2.5 Hz, 1 H), 1.74 (s, 3H), 1.78-1.68 (m, 2H), 1.64 (h, *J ₌* 6.8 Hz, 1 H), 1.52 (s, 3H), 1.42 (ddd, *J* = 12.0, 4.6, 2.6 Hz, 1H), 1.41-1.35 (m,1 H), 1.35-1.24 (m, 2H), 1.23-1.11 (m, 1H), 0.96 (s, 3H), 0.88 (s, 3H), 0.78 (d, *J* = 7.1 Hz, 3H) ppm.
¹³C-NMR (C₆D₆): 109.85, 84.90, 79.33, 78.73, 59.15, 58.73, 57.86, 52.52, 42.38, 42.32, 41.99, 38.74, 36.24, 31.14, 28.76, 28.11, 25.93, 25.63, 15.63 ppm.

### Geruch: schwach Ambra

### Beispiel 2

### Umsetzung von cis-Cedrandiol mit Methoxyacetaldehyddimethylacetal

24 g (0,1 Mol) cis-Cedrandiol (eingesetzt wurden 26 g mit einem GC-Gehalt von 92%), 127,6 g (1,45 mol) Methoxyacetaldehyddimethylacetal und 0,4 g konzentrierte Schwefelsäure wurden 24 Stunden bei 10°C gerührt. Nach dem Reaktionsende wurde Methyl-tert.-butylether zugegeben, mit einer 5%-igen Natriumhydrogencarbonat-Lösung gewaschen und die Leichtsieder abdestilliert. Nach Hinzufügen von n-Hexan kristallisierte nicht umgesetztes cis-Cedrandiol aus, das durch Filtration abgetrennt wurde. Nach Einengen des Filtrats wurden 30,4 g Rohprodukt erhalten. Gemäß GC-Analyse lag der Gehalt der Verbindungen der Formeln (X) und (XI) in dem erhaltenen Produkt bei insgesamt 51% (entsprechend 50% Ausbeute der Theorie). Durch Lösen des Rohproduktes in Dipropylenglykol (DPG) wurde hieraus eine Lösung mit einem Gehalt von 10 Gew.-% an Verbindungen der Formel (X) und (XI) (mit einem Verhältnis von (X) : (XI) = 1 : 6,2) hergestellt.

Für analytische Untersuchungen wurden aus dem Rohprodukt die stereoisomeren Verbindungen der Formel (X) und (XI) mittels Flüssigkeitschromatographie an Kieselgel 60 (0,04-0,063 mm) mit dem Elutionsmittel Cyclohexan/Essigsäureethylester (60/1) voneinander getrennt. Die Verbindung der Formel (X) wurde mit einer Reinheit von 99,4% und die Verbindung der Formel (XI) mit einer Reinheit von 96,9% erhalten.

### Verbindung der Formel (X):

MS: m/z: 41 (24), 43 (20), 45 (16), 55 (21), 69 (64), 93 (17), 95 (17), 105 (50), 119 (100), 133 (58), 147 (26), 203 (86), 249 (25)

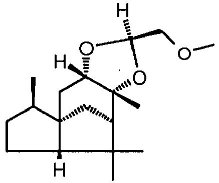

### Geruch: stark Ambra, Holz

### Verbindung der Formel (XI):

MS: m/z: 41 (23), 43 (19), 45 (16), 55 (23), 69 (60), 93 (17), 95 (17), 105 (49), 119 (100), 133 (57), 147 (24), 203 (83), 249 (26)

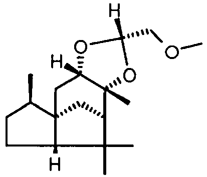

### Geruch: schwach Ambra

In den nachfolgenden Parfümölrezepturen und Anwendungsbeispielen wurden jeweils Lösungen mit dem angegebenen Gehalt eingesetzt, wobei das Gewichtsverhältnis von der Verbindung der Formel (VIII) zu der Verbindung der Formel (IX)) bei 3 : 8 und das Gewichtsverhältnis von (X) zu (XI) bei 3 : 8 lag.

### Erfindungsgemäßes Parfümöl-Beispiel P1:

| | Ref-1 | P1 |
|---|---|---|
| HEXENALDIMETHYLACETAL, 2,2,5-TRIMETHYL-4- | 10 | 10 |
| HEXADECEN-1,16-OLID, 7(9)Z- | 15 | 15 |
| CYCLOHEXADECENON, (8E/Z)- + CYCLOHEXADECENON, (7E)- | 140 | 140 |
| BUT-2-EN-1-ON, 1-(2,6,6-TRIMETHYL-CYCLOHEX-3-ENYL)- (E) 10%DPG | 10 | 10 |
| BRASSYLSAEUREETHANDIOLESTER | 120 | 120 |
| NONADIEN-3-OL, 3,7-DIMETHYL-1,6- | 30 | 30 |
| PYRANOL, 2-ISOBUTYL-4-METHYL-4-TETRAHYDRO- (E/Z) | 40 | 40 |
| BUTTERSAEURE-5-HEXENYLESTER, 2-METHYL- | 5 | 5 |
| ESSIGSAEUREGERANYLESTER | 40 | 40 |
| METHYL-DIHYDROJASMONAT | 400 | 400 |
| PROPIONSAEURE-2-(1-(3,3-DIMETHYLCYCLOHEXYL)-ETHOXY-2-METHYLPROPYLESTER | 30 | 30 |
| ESSIGSAEURE-3Z-HEXENYLESTER | 5 | 5 |
| SALICYLSAEURE-3Z-HEXENYLESTER | 20 | 20 |
| BICYCLO[4.4.0]DECEN, 3,4,10,10-TETRAMETHYL-3-HYDROXYETHYL-1 (6) | 50 | 50 |
| CYCLOHEXANON, 3,3,5,5-TETRAMETHYL-4-(1-ETHOXYVINYL)- | 10 | 10 |
| KOHLENSAEURE-3Z-HEXENYLMETHYLESTER, 10% in DPG | 10 | 10 |
| MANDARINENOEL | 20 | 20 |
| 2(3H)-FURANON, 5-HEXYL-DIHYDRO-4-METHYL-(E/Z) | 5 | 5 |
| ESSIGSAEUREPRENYLESTER | 5 | 5 |
| TRICYCLO(5.2.1.0)DECAN, 8-FORMYL- | 15 | 15 |
| DIOXOL, 4H-4A,9-METHANOAZULENO(5,6-D)-OCTAHYDRO-2,2,5,8,8,9A-HEXAMETHYL-, 1,3-(Ambrocenide®), 0,1% in DPG | 20 | 0 |
| (Verbindung der Formel (VIII)), 0,1% in DPG | 0 | 20 |
| TOTAL | 1000 | 1000 |

Das Vergleichsbeispiel (Ref-1) mit Ambrocenide® riecht frisch, fruchtig und transparent. In Parfümöl P1 verleiht die Verbindung der Formel (VIII) der Komposition eine Eleganz und ausgeprägtere Fruchtigkeit.

### Erfindungsgemäßes Parfümöl-Beispiel P2:

| | Ref-2 | P2 |
|---|---|---|
| UNDECALACTON, GAMMA- | 2 | 2 |
| HEXADECEN-1,16-OLID, 7(9)Z- | 10 | 10 |
| CYCLOHEXADECENON, (8E/Z)- + CYCLOHEXADECENON, | 100 | 100 |
| (7E)- | | |
| CARDAMOMENOEL CEYLON | 2 | 2 |
| INDAN-4-ON, 1,1,2,3,3-PENTAMETHYL-TETRAHYDRO- | 15 | 15 |
| CEDERNHOLZOEL VIRGINIA | 100 | 100 |
| DAMASCON, -BETA-, 10% in DPG | 2 | 2 |
| METHYL-DIHYDROJASMONAT | 210 | 210 |
| BICYCLO[4.4.0]DECEN, 3,4,10,10-TETRAMETHYL-3-HYDROXYETHYL-1 (6) | 300 | 300 |
| IRALDEIN, GAMMA- | 60 | 60 |
| CYCLOHEXANON, 3,3,5,5-TETRAMETHYL-4-(1-ETHOXYVINYL)- | 10 | 10 |
| CYCLOPENTADECENON, 3-METHYL-5E- | 20 | 20 |
| PATCHOULI OEL | 5 | 5 |
| PENTEN-2-OL, 3,3-DIMETHYL-5-(2,2,3-TRIMETHYL-3-CYCLOPENTENYL)-4- | 20 | 20 |
| PENTANOL, 3-METHYL-5-(2,2,3-TRIMETHYL-3-CYCLOPENTENYL)-2- + HEXANOL, 6-(2,2,3-TRIME-3-CYCLOPENTENYL)-3- | 80 | 80 |
| VANILLIN | 2 | 2 |
| ISOLONGIFOLANONETHANDIOLKETAL | 30 | 30 |
| ZIMTRINDENOEL CEYLON | 2 | 2 |
| DIOXOL, 4H-4A,9-METHANOAZULENO(5,6-D)-OCTAHYDRO-2,2,5,8,8,9A-HEXAMETHYL-, 1,3- (Ambrocenide®), 0,1% in DPG | 30 | 0 |
| (Verbindung der Formel (VIII)), 0,1% in DPG | 0 | 30 |
| TOTAL | 1000 | 1000 |

Das Vergleichsbeispiel (Ref-2) mit Ambrocenide® riecht blumig-würzig mit trockenen holzigen Akzenten. In Parfümöl P2 wird durch die Verbindung der Formel (VIII) die würzige Note unterstützt, so dass die gesamte Komposition pfeffriger und charaktervoller erscheint.

### Erfindungsgemäßes Parfümöl-Beispiel P3:

| | Ref-3 | P3 |
|---|---|---|
| HEXENALDIMETHYLACETAL, 2,2,5-TRIMETHYL-4- | 10 | 10 |
| BENZODIOXEPINON, 7-METHYL-3,4-DIHYDRO-3- | 10 | 10 |
| PROPANAL, 2-METHYL-3-(4-METHOXYPHENYL)- | 5 | 5 |
| CITRONELLOL, BETA- | 20 | 20 |
| CITRONENOEL | 15 | 15 |
| CYCLOHEXYLOXYESSIGSAEUREALLYLESTER, 10% in DPG | 10 | 10 |
| DAMASCON, TRANS-ALPHA-, 1% in DPG | 20 | 20 |
| BUT-2-EN-1-ON, 1-(2,6,6-TRIMETHYL-CYCLOHEX-3-ENYL)-(E), 10% in DPG | 5 | 5 |
| DECALACTON GAMMA, 10% in DPG | 15 | 15 |
| BRASSYLSAEUREETHANDIOLESTER | 80 | 80 |
| NONADIEN-3-OL, 3,7-DIMETHYL-1,6- | 70 | 70 |
| PHENOL, 2-METHOXY-4-(2-PROPENYL)- | 5 | 5 |
| PYRANOL, 2-ISOBUTYL-4-METHYL-4-TETRAHYDRO- (E/Z) | 80 | 80 |
| BUTTERSAEURE-5-HEXENYLESTER, 2-METHYL- | 15 | 15 |
| METHYL-DIHYDROJASMONAT | 250 | 250 |
| PROPANAL, 2-PIPERONYL- | 30 | 30 |
| SALICYLSAEURE-3Z-HEXENYLESTER | 5 | 5 |
| INDOL, 10% in DPG | 10 | 10 |
| IRALDEIN, GAMMA- | 30 | 30 |
| BICYCLO[4.4.0]DECEN, 3,4,10,10-TETRAMETHYL-3-HYDROXYETHYL-1 (6) | 80 | 80 |
| PENTADECANOLID, 1,15- | 20 | 20 |
| HEPTENAL, 2,6-DIMETHYL-5-, 10% in DPG | 3 | 3 |
| PYRIDIN, 4-DECYL-, 1% in DPG | 2 | 2 |
| CYCLOHEXEN, 2,4-DIMETHYL-1-FORMYL-3-, 10% in DPG | 10 | 10 |
| DIOXOL, 4H-4A,9-METHANOAZULENO(5,6-D)-OCTAHYDRO-2,2,5,8,8,9A-HEXAMETHYL-, 1,3- (Ambrocenide®), 0,1% in DPG | 5 | 0 |
| (Verbindung der Formel (X)), 0,1% in DPG | 0 | 5 |
| TOTAL | 805 | 805 |

Das Vergleichsbeispiel (Ref-3) mit Ambrocenide® besticht durch seine transparente Frische: In Parfümöl P3 wird durch die Verbindung der Formel (X) erreicht, dass die Komposition kosmetischer-pflegender mit mehr Volumen wahrgenommen wird.

### Erfindungsgemäßes Parfümöl-Beispiel P4:

| | Ref-4 | P4 |
|---|---|---|
| NAPHTHO[2.1-B]FURAN, DODECAHYDRO-3A.6.6.9A-TETRAMETHYL- | 5 | 5 |
| BENZOE SIAM ABS., 50% in DPG | 10 | 10 |
| SALICYLSAEUREBENZYLESTER | 20 | 20 |
| BERGAMOTTOEL | 50 | 50 |
| CEDRYLMETHYLETHER | 40 | 40 |
| CISTUS LABDANUM | 35 | 35 |
| CISTUSOEL SPAN., 10% in DPG | 10 | 10 |
| DAMASCON, TRANS-ALPHA-, 1 % in DPG | 20 | 20 |
| MYRCENOL, DIHYDRO- | 30 | 30 |
| BRASSYLSAEUREETHANDIOLESTER | 120 | 120 |
| BENZOESAEUREMETHYLESTER, 2,4-DIHYDROXY-3,6-DIMETHYL-, 1% in DPG | 20 | 20 |
| PYRANOL, 2-ISOBUTYL-4-METHYL-4-TETRAHDRO- (E/Z) | 20 | 20 |
| PENTADECEN-1,15-OLID, (11E/Z)-+ PENTADECEN-1,15-OLID, 12E- | 30 | 30 |
| METHYL-DIHYDROJASMONAT | 300 | 300 |
| BICYCLO[4.4.0]DECEN, 3,4,10,10-TETRAMETHYL-3-HYDROXYETHYL-1 (6) | 75 | 75 |
| ISOBUTYLCHINOLIN, 10% in DPG | 3 | 3 |
| CYCLOHEXANON, 3,3,5,5-TETRAMETHYL-4-(1-ETHOXYVINYL)- | 30 | 30 |
| LABDANUM ABSOLUE | 20 | 20 |
| ESSIGSAEURELINALYLESTER | 50 | 50 |
| MANDARINENOEL | 10 | 10 |
| CYCLOPENTADECENON, 3-METHYL-5E- | 10 | 10 |
| MUSKATELLER SALBEIOEL | 10 | 10 |
| OLIBANUM COEUR, 50% in TEC | 5 | 5 |
| PATCHOULIOEL | 20 | 20 |
| PYRIDIN, 4-DECYL-, 1% in DPG | 2 | 2 |
| BUTENOL, 2-ETHYL-4-(2,2,3-TRIMETHYL-3-CYCLOPENTENYL)-2E- | 5 | 5 |
| VANILLIN | 30 | 30 |
| DIOXOL, 4H-4A,9-METHANOAZULENO(5,6-D)-OCTAHYDRO-2,2,5,8,8,9A-HEXAMETHYL-, 1,3- (Ambrocenide®), 0,1% in DPG | 20 | 0 |
| (Verbindung der Formel (X)), 0,1% in DPG | 0 | 20 |
| TOTAL | 1000 | 1000 |

Das Vergleichsbeispiel (Ref-4) mit Ambrocenide® besticht durch eine schöne Holznote. In Parfümöl P4 verleiht die Verbindung der Formel (X) der Komposition mehr Volumen und die Tabaknote wird verstärkt.

### Erfindungsgemäßes Parfümöl-Beispiel P5:

| | Ref-5 | P5 |
|---|---|---|
| CYCLOHEXADECENON, (8E/Z)- + CYCLOHEXADECENON, (7E)- | 200 | 200 |
| BEIFUSSOEL | 10 | 10 |
| BERGAMOTTOEL | 60 | 60 |
| CEDRYLMETHYLETHER | 40 | 40 |
| CISTUSOEL SPAN., 10% in DPG | 10 | 10 |
| CITRONENOEL | 20 | 20 |
| CUMARIN | 40 | 40 |
| CYCLOHEXYLOXYESSIGSAEUREALLYLESTER, 10% in DPG | 5 | 5 |
| MYRCENOL, DIHYDRO- | 50 | 50 |
| BRASSYLSAEUREETHANDIOLESTER | 120 | 120 |
| BENZOESAEUREMETHYLESTER, 2,4-DIHYDROXY-3,6-DIMETHYL-, 10% in DPG | 20 | 20 |
| METHYL-DIHYDROJASMONAT | 30 | 30 |
| IRALDEIN, GAMMA- | 20 | 20 |
| BICYCLO[4.4.0]DECEN, 3,4,10,10-TETRAMETHYL-3-HYDROXYETHYL-1 (6) | 120 | 120 |
| KRAUSEMINZOEL, 10% in DPG | 10 | 10 |
| LAVENDELOEL | 10 | 10 |
| LINALOOL | 20 | 20 |
| ESSIGSAEURELINALYLESTER | 20 | 20 |
| INDENO[1.2-D]-M-DIOXIN, 2,4-DIMETHYL-TETRAHYDRO- | 90 | 90 |
| MUSKATELLER SALBEI ABSOLUE | 5 | 5 |
| NELKENBLUETENOEL | 10 | 10 |
| VANILLIN | 20 | 20 |
| DIOXOL, 4H-4A,9-METHANOAZULENO(5,6-D)-OCTAHYDRO-2,2,5,8,8,9A-HEXAMETHYL-, 1,3- (Ambrocenide®), 0,1% in DPG | 20 | 0 |
| (Verbindung der Formel (VIII)), 0,1% in DPG | 0 | 20 |
| TOTAL | 950 | 950 |

Das Vergleichsbeispiel (Ref-5) mit Ambrocenide® wirkt sehr strahlend und intensiv lavendelartig. In Parfümöl P5 wird durch die Verbindung der Formel (VIII) die gesamte Komposition natürlicher lavendelartig, und erscheint dadurch hochwertiger mit mehr Volumen.

### Erfindungsgemäßes Parfümöl-Beispiel P6:

| | Ref-6 | P6 |
|---|---|---|
| HEXADECEN-1,16-OLID, 7(9)Z- | 20 | 20 |
| CYCLOHEXADECENON, (8E/Z)- + CYCLOHEXADECENON, (7E)- | 200 | 200 |
| INDAN-4-ON, 1,1,2,3,3-PENTAMETHYL-TETRAHYDRO- | 20 | 20 |
| BRASSYLSAEUREETHANDIOLESTER | 150 | 150 |
| BUTTERSAEURE-5-HEXENYLESTER, 2-METHYL- | 5 | 5 |
| PENTADECEN-1,15-OLID, (11E/Z)-+ PENTADECEN-1,15-OLID, 12E- | 90 | 90 |
| METHYL-DIHYDROJASMONAT | 210 | 210 |
| BICYCLO[4.4.0]DECEN, 3,4,10,10-TETRAMETHYL-3-HYDROXYETHYL-1 (6) | 200 | 200 |
| CYCLOHEXANON, 3,3,5,5-TETRAMETHYL-4-(1-ETHOXYVINYL)- | 20 | 20 |
| PENTEN-2-OL, 3,3-DIMETHYL-5-(2,2,3-TRIMETHYL-3-CYCLOPENTENYL)-4- | 5 | 5 |
| ISOLONGIFOLANONETHANDIOLKETAL | 70 | 70 |
| DIOXOL, 4H-4A,9-METHANOAZULENO(5,6-D)-OCTAHYDRO-2,2,5,8,8,9A-HEXAMETHYL-, 1,3- (Ambrocenide®), 0,1% in DPG | 10 | 0 |
| (Verbindung der Formel (VIII)), 0,1% in DPG | 0 | 10 |
| TOTAL | 1000 | 1000 |

Das Vergleichsbeispiel (Ref-6) mit Ambrocenide® besitzt eine kühle Transparenz gepaart mit einer trockenen Holzigkeit. In Parfümöl P6 verleiht die Verbindung der Formel (VIII) der gesamten Komposition eine ausgeprägte Harmonie und Wärme, wobei die Fruchtigkeit verstärkt (gepusht) wird hin zu einer schönen Pflaumennote gepaart mit Tabakeffekten.

### Erfindungsgemäßes Parfümöl-Beispiel P7:

| | Ref-7 | P7 |
|---|---|---|
| NAPHTHO[2.1-B]FURAN, DODECAHYDRO-3A.6.6.9A-TETRAMETHYL- | 5 | 5 |
| SALICYLSAEUREBENZYLESTER | 50 | 50 |
| BERGAMOTTOEL | 50 | 50 |
| CUMARIN | 5 | 5 |
| DAMASCON, TRANS-ALPHA-, 1% in DPG | 20 | 20 |
| DECALACTON GAMMA, 10% in DPG | 15 | 15 |
| BRASSYLSAEUREETHANDIOLESTER | 30 | 30 |
| NONADIEN-3-OL, 3,7-DIMETHYL-1,6- | 60 | 60 |
| ESSIGSAEURE-3,7-DIME-1,6-NONADIEN-3-YLESTER | 50 | 50 |
| PYRANOL, 2-ISOBUTYL-4-METHYL-4-TETRAHYDRO- (E/Z) | 70 | 70 |
| BUTTERSAEURE-5-HEXENYLESTER, 2-METHYL- | 5 | 5 |
| BICYCLO[2.2.2]OCTEN, 6-ME-8-ISOPROPYL-2(3)-(1,3-DIOXOLAN-2-YL | 5 | 5 |
| METHYL-DIHYDROJASMONAT | 280 | 280 |
| PROPANAL, 2-PIPERONYL- | 20 | 20 |
| BENZALDEHYD, 3,4-METHYLENDIOXY- | 10 | 10 |
| PROPIONSAEURE-2-(1-(3,3-DIMETHYLCYCLOHEXYL)- | 60 | 60 |
| ETHOXY-2-METHYLPROPYLESTER | | |
| SALICYLSAEURE-3Z-HEXENYLESTER | 5 | 5 |
| BICYCLO[4.4.0]DECEN, 3,4,10,10-TETRAME-3-HYDROXYETHYL-1(6) | 120 | 120 |
| PENTADECANOLID, 1,15- | 30 | 30 |
| MANDARINENOEL | 20 | 20 |
| CYCLOPENTADECENON, 3-METHYL-5E- | 3 | 3 |
| BUTANOL, 2-METHYL-4-PHENYL-2- | 10 | 10 |
| PYRIDIN, 4-DECYL-, 1% in DPG | 2 | 2 |
| PENTEN-2-OL,3,3-DIMETHYL-5-(2,2,3-TRIMETHYL-3-CYCLOPENTENYL)-4- | 20 | 20 |
| CYCLOHEXANOL, 4-(3-METHYL-BUTYL)- (E/Z) | 15 | 15 |
| VANILLIN | 20 | 20 |
| DIOXOL, 4H-4A,9-METHANOAZULENO(5,6-D)-OCTAHYDRO-2,2,5,8,8,9A-HEXAMETHYL-, 1,3- (Ambrocenide®), 0,1% in DPG | 5 | 0 |
| (Verbindung der Formel (VIII)), 0,1% in DPG | 0 | 5 |
| DIPROPYLENGLYKOL | 15 | 15 |
| TOTAL | 1000 | 1000 |

Durch den Austausch von Ambrocenide® des Vergleichsbeispiel (Ref-7) durch die Verbindung der Formel (VIII) wirkt die gesamte Komposition P7 voluminöser, eleganter und somit auch hochwertiger, Parfümöl P7 wirkt komplexer und die Coumarin Note wird sehr schön harmonisiert.

### Erfindungsgemäßes Parfümöl-Beispiel P8:

| | Ref-8 | P8 |
|---|---|---|
| Ethylacetoacetat | 60 | 60 |
| Propanal, 2-Methyl-2-(4-ethylbenzyl)- | 25 | 25 |
| Cyclohexen, 2,4-Dimethyl-1-formyl-3- (E/Z) | 15 | 15 |
| Hexenol Cis-3 | 130 | 130 |
| Kohlensäure-3Z-hexenylmethylester | 20 | 20 |
| Cyclohexyloxyessigsäureallylester | 25 | 25 |
| Benzenmethanol, .alpha.-methyl-, 1-acetat | 40 | 40 |
| Linalylacetat | 80 | 80 |
| Myrcenol, Dihydro- | 650 | 650 |
| Mandarinenöl | 260 | 260 |
| Grapefruitöl | 260 | 260 |
| Hexenaldimethylacetal, 2,2,5-Trimethyl-4- | 170 | 170 |
| Methylanthranilat, 10% DPG | 155 | 155 |
| Cardamomöl | 5 | 5 |
| Red Berry Extract | 25 | 25 |
| Muskatnussöl | 20 | 20 |
| Ethylbutyrat, 10% DPG | 10 | 10 |
| Undecalacton, gamma- | 65 | 65 |
| Pentansäureethylester, 2-Methyl-, 10% DPG | 15 | 15 |
| Heptansäure, 2-propen-1-yl ester | 40 | 40 |
| Ethylmaltol | 15 | 15 |
| Benzenepropanal, alpha-Methyl-4-(1.1-dimethylethyl)- | 250 | 250 |
| 1.3-Benzodioxol-5-propanal, alpha-Methyl- | 200 | 200 |
| Hydroxycitronellal | 200 | 200 |
| 4-Methyl-2-(2-methylpropyl)oxan-4-ol | 260 | 260 |
| 1.6-Octadien-3-ol, 3.7-Dimethyl- | 40 | 40 |
| Nonadien-3-ol, 3,7-Dimethyl-1,6- | 130 | 130 |
| Buttersäure, 1,1-dimethyl-2-phenylethylester | 65 | 65 |
| Phenylethylalkohol | 40 | 40 |
| Propionsäure-2-phenoxyethylester, 2-Methyl- | 40 | 40 |
| 2-Buten-1-on, 1-(2.6.6-Trimethyl-2-cyclohexen-1-yl)- (E) | 40 | 40 |
| 2-Buten-1-on, 1-(2.6.6-Trimethyl-1.3-cyclohexadien-1-yl)- | 5 | 5 |
| Methyl-dihydrojasmonat | 390 | 390 |
| 3-Buten-2-on, 3-Methyl-4-(2.6.6-trimethyl-2-cyclohexen-1-yl)- | 220 | 220 |
| Vanillin | 230 | 230 |
| Cinnamon Bark Oil | 15 | 15 |
| 2H-1-Benzopyran-2-on | 200 | 200 |
| Cyclohexanolacetat, 4-(1.1-Dimethylethyl)- (Z) | 50 | 50 |
| Cedarwood Oil | 15 | 15 |
| Bicyclo[4.4.0]decen, 3,4,10,10-Tetramethyl-3-hydroxyethyl-1(6) | 1.500 | 1.500 |
| Cyclododecan, (Ethoxymethoxy)- | 300 | 300 |
| 2-Buten-1-ol, 2-Ethyl-4-(2.2.3-trimethyl-3-cyclopenten-1-yl)- (2E) | 130 | 130 |
| Dioxol, 4H-4a,9-Methanoazuleno(5,6-d)-octahydro-2,2,5,8,8,9a-hexamethyl-, 1,3- (Ambrocenide®), 10,0% in DPG | 30 | 0 |
| (Verbindung der Formel (X)), 10,0% in DPG | 0 | 30 |
| 5H-3.5a-Epoxynaphth[2.1-c]oxepin, Dodecahydro-3.8.8.11a-tetramethyl- | 1.200 | 1.200 |
| Naphtho[2.1-b]furan, Dodecahydro-3a.6.6.9a-tetramethyl- | 220 | 220 |
| Dipropylenglykol | 175 | 175 |
| TOTAL | 8.030 | 8.030 |

Das Vergleichsbeispiel Ref-8 mit Ambrocenide® zeichnet sich durch den Widerspruch zwischen frischen holzigen Noten und süßen balsamischen Aspekten aus. Der Austausch von Ambrocenide® durch die Verbindung der Formel (X) verleiht der gesamten Komposition mehr Impact, Volumen, Charakter gepaart mit samtigen-pudrigen Aspekten.

### Erfindungsgemäßes Parfümöl-Beispiel P9:

| | Ref-9 | P9 |
|---|---|---|
| Undecenal, 2,6,10-Trimethyl-9- | 15,00 | 15,00 |
| Propanal, 2-Methyl-2-(4-ethylbenzyl)- | 3,00 | 3,00 |
| Cis-3 Hexenylacetat | 3,00 | 3,00 |
| Cyclohexen, 2,4-Dimethyl-1-formyl-3- (E/Z) | 2,00 | 2,00 |
| Tricyclo(5.2.1.0)decan, 8-Formyl- | 2,00 | 2,00 |
| Cyclohexyloxyessigsäureallylester | 4,00 | 4,00 |
| Heptenal, 2,6-Dimethyl-5- | 2,00 | 2,00 |
| Identoil® Bergamot | 45,00 | 45,00 |
| Myrcenol, Dihydro- | 90,00 | 90,00 |
| 2.6-Octadienal, 3.7-Dimethyl- (2E/Z) | 10,00 | 10,00 |
| 2-Pentennitril, 3-Methyl-5-phenyl- (2E/Z) | 3,00 | 3,00 |
| Orangenöl | 30,00 | 30,00 |
| Hexenaldimethylacetal, 2,2,5-Trimethyl-4- | 2,00 | 2,00 |
| Lavandinöl | 10,00 | 10,00 |
| Cardamomöl | 1,00 | 1,00 |
| Pentansäureethylester, 2-Methyl- , 10% DPG | 10,00 | 10,00 |
| Benzenepropanal, alpha-Methyl-4-(1.1-dimethylethyl)- | 40,00 | 40,00 |
| 2H-1.5-Benzodioxepin-3(4H)-on, 7-Methyl- | 20,00 | 20,00 |
| Benzenepropanol, beta.beta.3-Trimethyl- | 40,00 | 40,00 |
| Geraniumöl | 2,00 | 2,00 |
| 3-Buten-2-on, 1-(2.4.4-Trimethyl-2-cyclohexen-1-yl)- (2E) | 10,00 | 10,00 |
| alpha-Hexylcin namaldehyd | 60,00 | 60,00 |
| Cyclopentanessigsäuremethylester, 3-Oxo-2-pentyl- (E) | 90,00 | 90,00 |
| Propannitril, 3-(3-Hexenyloxy)- (Z) | 2,00 | 2,00 |
| 3-Buten-2-on, 4-(2.6.6-Trimethyl-1-cyclohexen-1-yl)- | 5,00 | 5,00 |
| Benzol, 1-Methoxy-4-(1-propenyl)- (E) | 10,00 | 10,00 |
| Ethanon, 1-(2-Benzofuranyl)- | 2,00 | 2,00 |
| Cyclohexanolacetat, 4-(1.1-Dimethylethyl)- (Z) | 30,00 | 30,00 |
| Cedarwood Oil (Zedernholzöl) | 15,00 | 15,00 |
| Cyclohexanpropanol, alpha-Ethyl-2.2.6-trimethyl- (E/Z) | 30,00 | 30,00 |
| Bicyclo[4.4.0]decen, 3,4,10,10-Tetramethyl-3-hydroxyethyl-1(6) | 100,00 | 100,00 |
| Cyclododecan, (Ethoxymethoxy)- | 30,00 | 30,00 |
| Patchouliöl | 10,00 | 10,00 |
| 2-Buten-1-ol, 2-Ethyl-4-(2.2.3-trimethyl-3-cyclopenten-1-yl)- (2E) | 30,00 | 30,00 |
| Benzoesäuremethylester, 2,4-Dihydroxy-3,6-dimethyl- | 3,00 | 3,00 |
| Tetramethyl dodecahydro-3a,6,6,9a-naphtho(2,1-b)furan | 12,00 | 12,00 |
| Isolongifolanonethandiolketal | 50,00 | 50,00 |
| Dioxol, 4H-4a,9-Methanoazuleno(5,6-d)-octahydro-2,2,5,8,8,9a- | 2,00 | 0 |
| hexamethyl-, 1,3- (Ambrocenide®), 10,0% in DPG | | |
| (Verbindung der Formel (X)), 10,0% in DPG | 0 | 2,00 |
| Pentadecanolid, 1,15- | 30,00 | 30,00 |
| Cyclohexadecenon, 8E/Z- | 120,00 | 120,00 |
| Dipropylenglykol | 25,00 | 25,00 |
| TOTAL | 1.000,00 | 1.000,00 |

Das Vergleichsbeispiel Ref-9 mit Ambrocenide® ist ein moderner Fougere Typ mit einer wässrigen Citrus Kopfnote und einem holzigen Fond. Durch den Austausch von Ambrocenide® gegen die Verbindung der Formel (X) wird der weiße Holz, sowie Zedern- und Sandelholzaspekt verstärkt.

### Erfindungsgemäßes Parfümöl-Beispiele P10:

| | Ref-10 | P10 |
|---|---|---|
| Undecenal, 2,6,10-Trimethyl-9- | 8 | 8 |
| 10-Undecenal | 8 | 8 |
| Cyclohexen, 2,4-Dimethyl-1-formyl-3- (E/Z) | 5 | 5 |
| Kohlensäure-3Z-hexenylmethylester | 8 | 8 |
| Indeno[1.2-d]-1.3-dioxin, 4.4a.5.9b-Tetrahydro-2.4-Dimethyl- | 70 | 70 |
| Cyclohexanolacetat, 3.3.5-Trimethyl- | 20 | 20 |
| Orangenöl | 20 | 20 |
| Methyl Naphthyl Ketone Beta | 5 | 5 |
| Ethanon, 1-(3-Methyl-2-benzofuranyl)- | 2 | 2 |
| 2-Butanon, 4-(4-Hydroxyphenyl)- | 1 | 1 |
| Peach Base | 8 | 8 |
| Pyranol, 2-Isobutyl-4-methyl-4-tetrahydro-(E/Z) | 10 | 10 |
| 1.6-Octadien-3-ol, 3.7-Dimethyl- | 90 | 90 |
| 2H-Pyran, Tetrahydro-2-methyl-2-(2-methyl-1-propenyl)-(E/Z) | 3 | 3 |
| 2-Phenylethanol | 70 | 70 |
| Benzenpentanol, beta-Methyl- | 25 | 25 |
| 2.6-Octadien-1-olacetat, 3.7-Dimethyl- (2E/Z) | 30 | 30 |
| But-2-en-1-on, 1-(2,6,6-Trimethyl-cyclohex-3-enyl)- (E) | 3 | 3 |
| Benzoesäu repropylester | 10 | 10 |
| Methyl-dihydrojasmonat (E) | 70 | 70 |
| Amyl Salicylat N/Iso | 17 | 17 |
| Salicylsäurebenzylester | 80 | 80 |
| Cis-3 Hexenylsalicylat | 10 | 10 |
| Benzoesäurehexylester, 2-Hydroxy- | 33 | 33 |
| 3-Decen-5-ol, 4-Methyl- | 5 | 5 |
| Orris Base | 5 | 5 |
| 3-Buten-2-on, 3-Methyl-4-(2.6.6-trimethyl-2-cyclohexen-1-yl)- | 80 | 80 |
| Benzaldehyd, 3,4-Methylendioxy- | 10 | 10 |
| Vanillin | 4 | 4 |
| Zimtalkohol | 4 | 4 |
| 2H-1-Benzopyran-2-on | 8 | 8 |
| Acetylcedren | 70 | 70 |
| Cyclododecan, (Ethoxymethoxy)- | 40 | 40 |
| 2-Pentanon, 4-Cyclohexyl-4-methyl- | 8 | 8 |
| Patchouli Base | 37 | 37 |
| Patchouliöl | 33 | 33 |
| Dioxol, 4H-4a,9-Methanoazuleno(5,6-d)-octahydro-2,2,5,8,8,9a-hexamethyl-, 1,3- (Ambrocenide®), 1,0% in DPG | 20 | 0 |
| Verbindung der Formel (VIII) 10,0% in DPG | | 2 |
| Pentadecen-1,15-olid, (11 E/Z)- + Pentadecen-1,15-olid, 12E- | 40 | 40 |
| Triethylcitrat | 30 | 48 |
| TOTAL | 1000 | 1000 |

Das Vergleichsbeispiel Ref-10 mit Ambrocenide® ist ein floraler Chypre Typ mit einer aldehydigen Kopfnote, gepaart mit floralen Herznoten und abgerundet durch Patchouli und Moschus. Der Austausch von Ambrocenide® gegen die Verbindung der Formel (VIII) verleiht der gesamten Komposition einen pflegenderen, wärmeren und voluminöseren Charakter, zusätzlich wird die Haftung verbessert.

Die Parfümöle P1, P2, P3, P4, P5, P6, P7, P8, P9 und P10 aus den obigen Parfümöl-Beispielen wurden jeweils separat in die nachfolgenden Formulierungen eingearbeitet.

Die oben bei dem jeweiligen Parfümöl beschriebenen geruchlichen Effekte wurden jeweils auch in den nachfolgenden Formulierungen beobachtet.

### Formulierungsbeispiele:

### Beispiel F1 - Waschpulver

| **Material** | **Chemischer Name** | **Funktion** | **Gew.-%** | **Gew.-%** |
|---|---|---|---|---|
| Natrium Metasilicat Pentahydrat | Sodium Metasilicate Pentahydrate | | Ad 100 | Ad 100 |
| Natrium hydrogencarbonat | Sodium hydrogen carbonate | Alkali | 15,0 | 15,0 |
| Natriumpercarbonat | Sodium carbonate peroxyhydrate | Bleichmittel | 15,0 | 15,0 |
| Peractive AC Blue | TAED / Na-Carboxymethylcellulose | Aktivator | 5,00 | 5,00 |
| Genapol OA-080 | Oxoalkohol C14-15, 8EO | Nichtionisches Tensid | 3,00 | 3,00 |
| Texapon K12 Pulver | Sodium Lauryl Sulphate C12 | Anionisches Tensid | 7,00 | 7,00 |
| Tinopal CBS-X | | Aufheller | 0,50 | 0,50 |
| Savinase 6.0 T, Type W | Protease | Enzym | 0,40 | 0,40 |
| Termamyl 120 T | Alpha-Amylase | Enzym | 0,30 | 0,30 |
| Natriumsulfat | Sodium Sulphate | Füllstoff | 5,50 | 5,50 |
| Parfumöl P1, P2, P3, P4, P5, P6, P7, P8, P9 bzw. | | Parfum (Fragrance) | 0,30 | 0,50 |
| P10 | | | | |

### Beispiel F2 - Allzweckreiniger

| **Material** | **Chemischer Name** | **Funktion** | **Gew.-%** | **Gew.-%** |
|---|---|---|---|---|
| Entionisiertes Wasser | Water | Lösungsmittel | Ad 100 | Ad 100 |
| Mergal K9N | 5-Chloro-2-methyl-3-(2H)-isothiazolone und 2-methyl-3-(2H)-isothiazolone | Konservierungsmittel | 0,1 | 0,1 |
| Trinatriumcitrat Dihydrat | Tri Sodium Citrate Dihydrate | Komplexbildner | 3,0 | 3,0 |
| Zetesol NL-2 | Fatty alcohol C12-14-sulfate, Sodium | Anionisches Tensid | 30,0 | 30,0 |
| Imbentin C/125/055 | Fatty alcohol C12-C15, 8EO | Nichtionisches Tensid | 5,0 | 5,0 |
| Ethanol | Ethanol | Lösungsmittel | 2,0 | 2,0 |
| Parfumöl P1, P2, P3, P4, P5, P6, P7, P8, P9 bzw. P10 | | Parfums (Fragrance) | 0,3 | 0,5 |

### Beispiel F3 - Shampoo

| **Material** | **INCI-Name** | **Gew.-%** | **Gew.-%** |
|---|---|---|---|
| Entionisiertes Wasser | Water | Ad 100 | Ad 100 |
| Plantacare PS 10 | Sodium Laureth Sulfate, Lauryl Glucoside | 20,0 | 20,0 |
| Euperlan PK 771 | Glycol Distearate, Sodium Lauryl Sulfate, Cocamide MEA, Laureth-10 | 6,0 | 6,0 |
| Dragocid Liquid | Phenoxyethanol, Methylparaben, Ethylparaben, Butylparaben, Propylparaben, Isobutylparaben | 0,5 | 0,5 |
| Natrium Chlorid | Sodium Chloride | 1,4 | 1,4 |
| Citronensäure Monohydrat kristallin | Citric Acid | 0,1 | 0,1 |
| Parfumöl P1, P2, P3, P4, P5, P6, P7, P8, P9 bzw. P10 | Parfum (Fragrance) | 0,5 | 0,8 |

### Beispiel F4 - Duschgel

| **Material** | **INCI-Name** | **Gew.-%** | **Gew.-%** |
|---|---|---|---|
| Entionisiertes Wasser | Wasser | Ad 100 | Ad 100 |
| Plantacare PS 10 | Sodium Laureth Sulfate, Lauryl Glucoside | 20,0 | 20,0 |
| Dragocid Liquid | Phenoxyethanol, Methyl-paraben, Ethylparaben, Butylparaben, Propylparaben, Isobutylparaben | 0,5 | 0,5 |
| Natriumchlorid | Sodium Chloride | 1,4 | 1,4 |
| Citronensäure Monohydrat kristallin | Citric Acid | 1,3 | 1,3 |
| Parfumöl P1, P2, P3, P4, P5, P6, P7, P8, P9 bzw. P10 | Parfum (Fragrance) | 0,5 | 0,7 |

### Beispiel F5 - Weichspüler

| **Material** | **Chemischer Name** | **Funktion** | **Gew.-%** | **Gew.-%** |
|---|---|---|---|---|
| Entionisiertes Wasser | Wasser | Lösungsmittel | Ad 100 | Ad 100 |
| Rewoquat WE 18 | Dialkylesterammoniumethosulfate | Kationisches Tensid | 16,6 | 16,6 |
| Mergal K9N | 5-Chloro-2-methyl-3-(2H)-isothiazolone und 2-methyl-3-(2H)-isothiazolone | Konservierungsmittel | 0,10 | 0,10 |
| Dow Corning 1520 Antifoam | Polydimethyl-siloxane | Entschäumer | 0,30 | 0,30 |
| Magnesium Chlorid 1 %ige Lösung | Magnesium Chlorid Lösung | Konsistenzgeber | 10,00 | 10,00 |
| Parfumöl P1, P2, P3, P4, P5, P6, P7, P8, P9 bzw. P10 | | Fragrance | 0,55 | 0,75 |

### Beispiel F6 - Eau de Cologne / Eau de Toilette

| **Inhaltsstoffe** | **Gew.-%** | **Gew.-%** |
|---|---|---|
| Parfumöl P1, P2, P3, P4, P5, P6, P7, P8, P9 bzw. P10 | 5 | 10 |
| Ethanol | Ad 100 | Ad 100 |
| Wasser | 18 | 10 |

### Beispiel F7 - Aerosol-Pumpspray

| **Inhaltsstoffe** | **Gew.-%** | **Gew.-%** |
|---|---|---|
| Parfumöl P1, P2, P3, P4, P5, P6, P7, P8, P9 bzw. P10 | 1,0 | 1,5 |
| Ethanol | Ad 100 | Ad 100 |
| Wasser | 5,0 | 8,0 |
| Alpha-Tocopherol | 0,20 | 0,20 |
| Hydroxypropylcellulose | 0,20 | - |
| Rosmarinextrakt, in Ethanol löslich | 0,22 | - |
| Cetylalkohol | 1,00 | 0,50 |

### Beispiel F8 - Shampoo

| **Inhaltsstoffe** | **Gew.-%** | **Gew.-%** | **Gew.-%** |
|---|---|---|---|
| Natriumlaurylethersulfat (z.B. Texapon NSO, Fa. Cognis Deutschland GmbH) | 12 | 12 | 12 |
| Cocamidopropylbetain (z.B. Dehyton K, Fa. Cognis Deutschland GmbH) | 2 | 2 | 2 |
| Natriumchlorid | 1,4 | 1,4 | 1,4 |
| Citronensäure | 1,3 | 1,3 | 1,3 |
| Phenoxyethanol, Methyl-, Ethyl-, Butyl- und Propylparaben | 0,5 | 0,5 | 0,5 |
| Parfumöl P1, P2, P3, P4, P5, P6, P7, P8, P9 bzw. P10 | 0,3 | 0,5 | 0,7 |
| Wasser | Ad 100 | Ad 100 | Ad 100 |

### Beispiel F9 - Waschpulver

| **Inhaltsstoffe** | **Gew.-%** | **Gew.-%** | **Gew.-%** |
|---|---|---|---|
| Lineares Na-Alkylbenzolsulfonat | 8,8 | 8,8 | 8,8 |
| Ethoxylierter Fettalkohol C12-18 (7 EO) | 4,7 | 4,7 | 4,7 |
| Na-Seife | 3,2 | 3,2 | 3,2 |
| Entschäumer DOW CORNING(R) 2-4248S POWDERED ANTIFOAM, Silikonöl auf Zeolith als Trägermaterial | 3,9 | 3,9 | 3,9 |
| Zeolith 4A Zeolith 4A | Ad 100 | Ad 100 | Ad 100 |
| Na-Carbonat | 11,6 | 11,6 | 11,6 |
| Na-Salz eines Copolymers aus Acryl- und Maleinsäure (Sokalan CP5) | 2,4 | 2,4 | 2,4 |
| Na-Silicat | 3,0 | 3,0 | 3,0 |
| Carboxymethylcellulose | 1,2 | 1,2 | 1,2 |
| Dequest 2066([[(Phosphonomethyl)imino]bis[(ethylennitrilo)bis (methylen)]]tetrakis-phosphonsäure, Natriumsalz) | 2,8 | 2,8 | 2,8 |
| Optischer Aufheller | 0,2 | 0,2 | 0,2 |
| Na-Sulfat | 6,5 | 6,5 | 6,5 |
| Protease | 0,4 | 0,4 | 0,4 |
| Natriumperborat tetrahydrat | 21,7 | 21,7 | 21,7 |
| Parfumöl P1, P2, P3, P4, P5, P6, P7, P8, P9 bzw. P10 | 0,25 | 0,35 | 0,5 |
| EDTA | 1,0 | 1,0 | 1,0 |

### Beispiel F10 - Flüssipwaschmittel

| **Inhaltsstoffe** | **Gew.-%** |
|---|---|
| Entionisiertes Wasser | 39,9 |
| Optischer Aufheller | 0,10 |
| Kokos Fettsäuren (C12-C18) | 7,5 |
| Kaliumhydroxid 50%ige Lösung | 4,3 |
| Propan-1,2-diol | 5,00 |
| Fettalkohole C12-C15, 8 EO | 12,0 |
| Na-Salz sekundärer Alkylsulfonate (C13-C17) | 17,0 |
| Triethanolamin | 2,0 |
| Trinatriumcitrat Dihydrat | 5,0 |
| Dequest 2066([[(Phosphonomethyl)imino]bis[(ethylennitrilo)bis (methylen)]]tetrakis-phosphonsäure, Natriumsalz) | 3,0 |
| Ethanol | 3,0 |
| Enzyme | 0,7 |
| Parfumöl P1, P2, P3, P4, P5, P6, P7, P8, P9 bzw. P10 | 0,5 |

### Beispiel F11 - Flüssigwaschmittel Konzentrat

| **Inhaltsstoffe** | **Gew**.-% |
|---|---|
| Entionisiertes Wasser | 13,4 |
| Kokos Fettsäuren (C12-C18) | 10,0 |
| Fettalkohole C12-C15, 8 EO | 26,0 |
| Na-Salz sekundärer Alkylsulfonate (C13-C17) | 26,5 |
| Triethanolamin | 8,5 |
| Na-Salz von Fettalkoholsulfaten C12-C14 | 3,0 |
| Ethanol | 5,5 |
| Harnstoff | 4,5 |
| Enzyme | 0,9 |
| Citronensäure | 1,0 |
| Parfumöl P1, P2, P3, P4, P5, P6, P7, P8, P9 bzw. P10 | 0,7 |

### Anwendungstechnische Beispiele:

### Beispiel Eigenhaftung: Verbindung der Formel (VIII) versus Ambrocenide®:

Der jeweils zu untersuchende Riechstoff wird als 10%ige Lösung in DPG auf codierte Riechstreifen getaucht, d.h. der zu untersuchende Riechstoff wird durch Eintauchen des jeweiligen Riechstreifens in die jeweilige Lösung des zu untersuchenden Riechstoffs auf den Riechstreifen aufgebracht, und unmittelbar anschließend in folgenden zeitlichen Abständen geruchlich bewertet:
1 Stunde; 3 Stunden; 10 Stunden; 1 Tag; 3 Tage; 10 Tage; 1 Monat; 3 Monate. Die Probanden bewerten die Geruchsintensität auf einer Skala von 1 = geruchlos bis 9 = sehr stark.

Getestet wird solange bis der Intensitäts-Mittelwert der Prüfer unter den Wert 1,5 (sehr schwach) fällt. Vergeben 50 % der Probanden eine Intensität von 1, und machen damit deutlich, dass der auf dem Riechstreifen getauchte Riechstoff nicht mehr wahrgenommen wird, wird der Test für beendet erklärt. Längstenfalls wird für 3 Monate getestet.

| | Verbindung der Formel (VIII), 10% in DPG | Ambrocenide®, 10 % in DPG |
|---|---|---|
| Eigenhaftung | mehr als 3 Monate | 10 Tage |

Die Verbindung der Formel (VIII) zeigt eine deutlich längere Haftung am Riechstreifen als Ambrocenide®.

### Beispiel Substantivität auf Haar: Verbindung der Formel (VIIII) versus Ambrocenide®:

Der zu untersuchende Riechstoff wird in einer Menge von 0,6 Gew.-% als 50%ige Lösung in DPG in eine Shampoo-Formulierung analog zu Beispiel F8 anstelle des dort eingesetzten Parfümöls eingearbeitet. Für jeden zu untersuchenden Riechstoff werden zwei Haarsträhnen benötigt. Außerdem wird als Referenz jeweils ein Haarsträhnenpärchen mit der unparfümierten Shampoo-Formulierung von Beispiel F8 gewaschen.

Alle Haarsträhnen werden zusammen in einem 2 I Becherglas mit Neutralshampoo gewaschen (mind. 2 h einweichen). Dann werden die Strähnen unter fließendem Wasser gut ausgespült und dann bei Raumtemperatur (ca. 23°C) getrocknet. Es werden von jedem Shampoo 100 ml einer 20 %igen wässrigen Lösung hergestellt (ebenso unparfümierte Masse). Nun wird je Shampoo ein Strähnenpaar gemeinsam für 2 min. in der Lösung gewaschen. Anschließend werden diese zwei Strähnen gemeinsam 20 sec. unter fließendem, handwarmem Wasser gespült. Beide Haarsträhnen werden gekämmt. Eine Haarsträhne wird nass in Alufolie eingewickelt. Die zweite Haarsträhne wird mit einem Haartrockner (Fön) getrocknet.

Ein Probandenpanel bewertet die vorbereiteten Haarsträhnen durch Riechen. Die Probanden bewerten die Intensität der Proben auf einer Skala von 0 (geruchlos) bis 6 (sehr stark).

### Reihenfolge:

a) frisch gewaschenes, trockenes Haar
b) frisch gewaschenes Haar, nass

| Substantivität auf Haar | Verbindung der Formel (VIII) | Ambrocenide® |
|---|---|---|
| Nasses Haar | 1,4 | 1,2 |
| Trockenes Haar | 1,7 | 0,5 |

Der Intensitätswert der Verbindung der Formel (VIII) ist auf trockenem Haar um mehr als den Faktor 3 höher verglichen mit Ambrocenide®.

### Beispiel Substantivität auf Baumwolle: Verbindung der Formel (VIII) versus Ambrocenide®:

Der zu untersuchende Riechstoff wird in einer Menge von 0,5 Gew.-% als 50%ige Lösung in DPG in eine Weichspüler-Formulierung analog zu Beispiel F5 anstelle des dort eingesetzten Parfümöls eingearbeitet.

Baumwolllappen werden bei 95 °C in einer Waschmaschine neutral gewaschen und bei Raumtemperatur offen getrocknet.

Jeweils zwei gleich codierte Baumwolllappen werden mit 370 g einer 1%igen wässrigen Weichspülerlauge enthaltend den jeweils zu untersuchenden Riechstoff in einem Topf der Linitest - Maschine im Weichspülprogramm gespült. Anschließend wird die Lauge der einzelnen Töpfe abgegossen, die Lappen pro Topf ausgewrungen und alle Lappenpaare als Paar 20 sec. ausgeschleudert. Dann wird von jedem Lappenpaar ein Lappen nass eingeschweißt, und einer zum Trocknen aufgehängt.

Ein Probandenpanel bewertet die vorbereiteten Proben durch Riechen.

Die Probanden bewerten die Intensität der Proben auf einer Skala von 0 (geruchlos) bis 6 (sehr stark)

Begonnen wird jeweils mit dem unparfümierten Muster und dann die Proben.

### Reihenfolge:

a) trockene Wäsche
b) nasse Wäsche

| Substantivität auf Baumwolle | Verbindung der Formel (VIII) | Ambrocenide® |
|---|---|---|
| Nasse Wäsche | 1,6 | 1,2 |
| Trockene Wäsche | 1,5 | 0,8 |

Der Intensitätswert der Verbindung der Formel (VIII) ist auf trockener Wäsche um den Faktor 2 höher verglichen mit Ambrocenide®, und auch die Intensität von nasser Wäsche wird für die Verbindung der Formel (VIII) höher bewertet.

Darüber hinaus wurden die trockenen Baumwolllappen über einen längeren Zeitraum gelagert und jeweils nach mehreren Tagen wie oben beschrieben bewertet.

| Tag 3 | Verbindung der Formel (VIII) | 2,00 |
|---|---|---|
| | Ambrocenide® | 1,20 |
| Tag 8 | Verbindung der Formel (VIII) | 1,79 |
| | Ambrocenide® | 0,93 |
| Tag 10 | Verbindung der Formel (VIII) | 1,80 |
| | Ambrocenide® | 1,10 |
| Tag 14 | Verbindung der Formel (VIII) | 1,69 |
| | Ambrocenide® | 0,92 |
| Tag 18 | Verbindung der Formel (VIII) | 1,80 |
| | Ambrocenide® | 0,50 |

Die Intensität der Verbindung der Formel (VIII) wird auch nach einem Zeitraum von 18 Tagen immer noch als deutlich stärker beurteilt verglichen mit Ambrocenide®.

## Patentansprüche

1. Verbindung der Formel (II) oder (II') 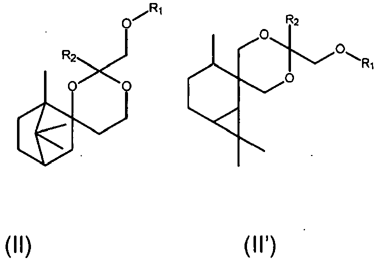 wobei R₁ Wasserstoff oder C₁-C₃-Alkyl, und R₂ Wasserstoff oder C₁-C₄-Alkyl oder der Verbindung der Formel (III) 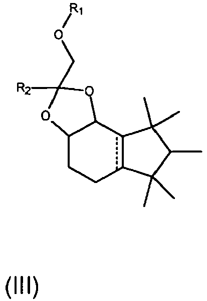 wobei R₁ Wasserstoff oder C₁-C₃-Alkyl, und R₂ Wasserstoff oder C₁-C₄-Alkyl ist bzw. sind, und die Verbindung gesättigt oder ungesättigt ist, oder
der Formel (IV) oder (IV') 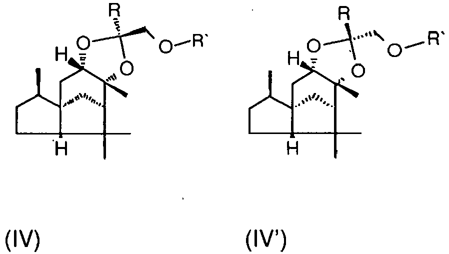 wobei gilt: R = H oder C₁-C₄-Alkyl und R'= H oder C₁-C₃-Alkyl.

2. Zusammensetzung umfassend oder bestehend aus
(i) ein(em) oder mehrere(n) 3,6,8,8-Tetramethyl-octahydro-3a,7-methanoazulen-5,6-diolketal(en) und
(ii) eine(r) oder mehrere(n) Verbindung(en) der Formel (II), (II'),(III), (IV) und/oder (IV') gemäß Anspruch 1,
**dadurch gekennzeichnet, dass**
- der Gesamtanteil der Verbindungen der Formel (II), (II'), (III), (IV) und/oder (IV') größer als 40 Gew bezogen auf die Gesamtmenge der in der Zusammensetzung enthaltenen 3,6,8,8-Tetramethyl-octahydro-3a,7-methano-azulen-5,6-diolketale ist,
und/oder dass
- das Gewichtsverhältnis der Verbindung(en) der Formel (IV) zu der/den Verbindung(en) der Formel (IV'), sofern vorhanden, im Bereich von 4 : 1 bis 1 : 10 liegt.

3. Verwendung einer Verbindung der Formel (II), (II'), (III) oder (IV) gemäß Anspruch 1 oder einer Zusammensetzung gemäß Anspruch 2 als
- Riechstoff bzw. Riechstoffmischung, mit den Geruchsnoten Ambra und/oder Holz,
- Mittel zum Erhöhen der Substantivität und/oder der Retention einer Riechstoffmischung, oder
- Fixateur.

4. Riechstoffmischung, umfassend oder bestehend aus
- ein(em) oder mehrere(n) Verbindung(en) der Formel (II), (II'), (III) oder (IV) gemäß Anspruch 1, oder eine(r) Zusammensetzung gemäß Anspruch 2
sowie
- einen/m oder mehrere(n) (weiteren) nicht der Formel (II), (II'), (III) oder (IV) entsprechenden Riechstoff(en),
**dadurch gekennzeichnet, dass**
(i) die Menge der Verbindung(en) der Formel (II), (II'), (III) oder (IV), ausreicht, eine Ambranote und/oder Holznote, zu vermitteln,
und/oder dass
(ii) der/die (weiteren) nicht der Formel (II), (II`), (III) oder (IV) entsprechenden Riechstoff(e) eine, mehrere oder sämtliche der Noten fruchtig, blumig, würzig, holzig, moschus und ambriert vermitteln und die Menge der Verbindung(en) der Formel (II), (II'), (III) oder (IV), ausreicht, eine, mehrere oder sämtliche der Noten fruchtig, blumig, würzig, holzig, moschus und ambriert zu modifizieren und/oder zu verstärken,
und/oder dass
(iii) die Menge der Verbindung(en) der Formel (II), (II'), (III) oder (IV), ausreicht, der Riechstoffmischung einen Eindruck von Volumen, Komplexität, Eleganz und/oder Natürlichkeit zu verleihen,
und/oder dass
(iv) die Menge der Verbindung(en) der Formel (II), (II'), (III) oder (IV), ausreicht, um im Vergleich zu einer ansonsten identisch zusammengesetzten Riechstoffmischung ohne Verbindung(en) der Formel (II), (II'), (III) oder (IV) einen pflegenderen, harmonischeren, hochwertigeren und/oder natürlicheren Geruchseindruck zu bewirken.

5. Riechstoffmischung, gemäß Anspruch 4, **dadurch gekennzeichnet, dass** die Menge der Verbindung(en) der Formel (II), (II'), (III) oder (IV), im Bereich von 0,0001 bis 25 Gew.-%, bezogen auf das Gesamtgewicht der Riechstoffmischung liegt.

6. Riechstoffmischung, gemäß Anspruch 4 oder 5, **dadurch gekennzeichnet, dass** die Riechstoffmischung
(i) zudem einen oder mehrere nicht der Formel (II), (II'), (III) oder (IV) entsprechende(n) Holz- und/oder Ambra-Riechstoff(e) und/oder
(ii) einen oder mehrere Moschusriechstoff(e) enthält.

7. Riechstoffmischung, gemäß Anspruch 6, **dadurch gekennzeichnet, dass** der/die Moschusriechstoff(e) ausgewählt ist/sind aus der Gruppe bestehend aus macrocyclischen Moschusriechstoffen, Nitro-Moschusriechstoffen, polycyclischen Moschusriechstoffen, alicyclischen Moschusriechstoffen und Kombinationen derselben.

8. Riechstoffmischung, gemäß einem oder mehreren der Ansprüche 6 bis 7, **dadurch gekennzeichnet, dass** das Gewichtsverhältnis an Moschusriechstoffen zu der/den Verbindung(en) der Formel (II), (II'), (III) oder (IV) im Bereich von 100 : 1 bis 100.000 : 1 liegt.

9. Verfahren zum Verstärken eines Geruchs oder mehrerer Gerüche ausgewählt aus der Gruppe bestehend aus fruchtig, blumig, würzig, holzig, moschus und ambriert, umfassend den folgenden Schritt:
(i) Vermischen von einem oder mehreren Riechstoff/en mit einer, mehreren oder sämtlichen der Noten ausgewählt aus der Gruppe bestehend aus fruchtig, blumig, würzig, holzig, moschus und ambriert mit einer oder mehreren Verbindung(en) der Formel (II), (II'), (III) oder (IV) gemäß Anspruch 1, einer Zusammensetzung gemäß Anspruch 2 oder einer Riechstoffmischung, gemäß einem oder mehreren der Ansprüche 4 bis 8, in einer Menge, die ausreicht, den Geruchseindruck der/des Riechstoffe(s) zu verstärken.

10. Parfümiertes Produkt enthaltend eine oder mehrere Verbindung(en) der Formel (II), (II'), (III) oder (IV) gemäß Anspruch 1, eine Zusammensetzung gemäß Anspruch 2 oder eine Riechstoffmischung gemäß einem oder mehreren der Ansprüche 4 bis 8, in einer sensorisch wirksamen Menge, wobei das Produkt ein oder mehrere Tenside enthält.

11. Parfümiertes Produkt gemäß Anspruch 10, **dadurch gekennzeichnet, dass** das Produkt ausgewählt ist aus der Gruppe bestehend aus Waschmitteln, Reinigungsmitteln, Hygienemitteln und Pflegemitteln, zur Anwendung im Bereich der Körperpflege, der Haarpflege, der Kosmetik oder des Haushalts.

12. Verfahren zum Versehen von Haaren oder textilen Fasern mit einer, mehrerer oder sämtlichen der Noten ausgewählt aus der Gruppe bestehend aus fruchtig, blumig, würzig, holzig, moschus und ambriert, umfassend die folgenden Schritte:
(i) Bereitstellen einer Riechstoffmischung gemäß einem der Ansprüche 4 bis 8, wobei der/die (weiteren) nicht der Formel (II), (II'), (III) oder (IV) entsprechenden Riechstoff(e) eine, mehrere oder sämtliche der Noten fruchtig, blumig, würzig, holzig, moschus und ambriert vermitteln und die Menge der Verbindung(en) der Formel (II), (II'), (III) oder (IV), ausreicht, um eine, mehrere oder sämtliche der Noten fruchtig, blumig, würzig, holzig, moschus und ambriert zu modifizieren und/oder zu verstärken, und
(ii) Applizieren der Riechstoffmischung auf das Haar oder die textilen Fasern.

## Claims

1. Compound of the formula (II) or (II') 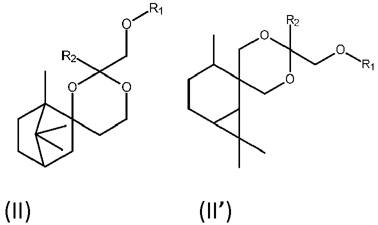 wherein R₁ is hydrogen or C₁-C₃ alkyl, and R₂ hydrogen or C₁-C₄ alkyl or a compound of the formula (III) 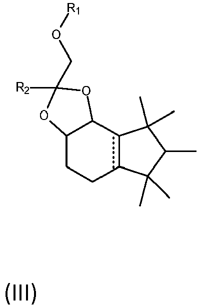 wherein R₁ is hydrogen or C₁-C₃ alkyl and R₂ is hydrogen or C₁-C₄ alkyl and the compound is saturated or unsaturated or
the formula (IV) or (IV') 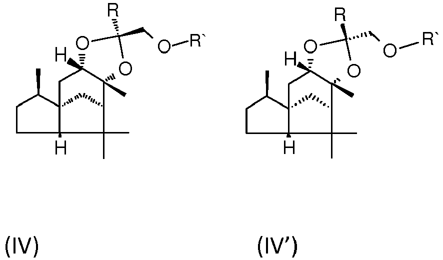 wherein: R = H or C₁-C₄ alkyl and R'= H or C₁-C₃ alkyl.

2. Composition comprising or consisting of
(i) one or more 3,6,8,8-tetramethyl-octahydro-3a,7-methano-azulen-5,6-diol ketal(s) and
(ii) one or more compound(s) of the formula (II), (II'), (III), (IV) and/or (IV') according to claim 1,
**characterized in that**
- the total content of compounds of the formula (II), (II'), (III), (IV) and/or (IV') is greater than 40 wt.% based on the total amount of 3,6,8,8-tetramethyl-octahydro-3a,7-methano-azulen-5,6-diol ketal(s) contained in the composition,
and/orthat
- the weight ratio of compound(s) of the formula (IV) to compound(s) of the formula (IV'), if present, lies in the range from 4 : 1 to 1 : 10.

3. The use of a compound of the formula (II), (II'), (III) or (IV) according to claim 1 or a composition according to claim 2 as
- fragrance or fragrance mixture, with the fragrance notes ambergris and/or wood,
- means for increasing the substantivity and/or retention of a fragrance mixture, or
- fixative.

4. Fragrance mixture comprising or consisting of
- one or more compound(s) of the formula (II), (II'), (III) or (IV) according to claim 1 or a composition according to claim 2
as well as
- one or more (further) compound(s) not corresponding to the fragrances of the formula (II), (II'), (III) or (IV),
**characterized in that**
(i) the quantity of compound(s) of the formula (II), (II'), (III) or (IV) is sufficient to mediate an ambergris and/or wood fragrance note,
and/orthat
(ii) the (further) fragrances not corresponding to the formula (II), (II'), (III) or (IV) mediate one, several or all of the notes fruity, floral, spicy, woody, musk and ambrette and the quantity of compound(s) of the formula (II), (II'), (III) or (IV) is sufficient to modify and/or intensify one, several or all the notes fruity, floral, spicy, woody, musk and ambrette,
and/orthat
(iii) the quantity of compound(s) of the formula (II), (II'), (III) or (IV) is sufficient to impart to the fragrance mixture an impression of volume, complexity, elegance and/or naturalness,
and/orthat
(iv) the quantity of compound(s) of the formula (II), (II'), (III) or (IV) is sufficient, in comparison to an otherwise identically constituted fragrance mixture without compounds of the formula (II), (II'), (III) or (IV), to create a more nurturing, more harmonious, higher quality and/or more natural olfactory impression.

5. The fragrance mixture according to claim 4, **characterized in that** the quantity of the compound(s) of the formula (II), (II'), (III) or (IV) lies in the range from 0,0001 to 25 wt.%, based on the total weight of the fragrance mixture.

6. The fragrance mixture according to claim 4 or 5, **characterized in that** the fragrance mixture
(i) additionally contains one or more wood and/or ambergris fragrance(s) not corresponding to the formula (II), (II'), (III) or (IV) and/or
(ii) contains one or more musk fragrance(s).

7. The fragrance mixture according to claim 6, **characterized in that** the musk fragrance(s) is/are selected from the group consisting of macrocyclic musk fragrances, nitro musk fragrances, polycyclic musk fragrances, alicyclic musk fragrances and mixtures thereof.

8. The fragrance mixture according to any one or more of the claims 6 to 7, **characterized in that** the weight ratio of musk fragrances to the compound(s) of the formula (II), (II'), (III) or (IV) lies in the range from 100 : 1 to 100.000 : 1.

9. Method for intensifying an odor or several odors selected from the group consisting of fruity, floral, spicy, woody, musk and ambrette, comprising the following steps:
(i) mixing of one or more fragrance(s) with one, several or all of the notes selected from the group consisting of fruity, floral, spicy, woody, musk and ambrette with one or more compound(s) of the formula (II), (II'), (III) or (IV) according to claim 1, of a composition according to claim 2 or a fragrance mixture according to one or more of claims 4 to 8, in a quantity which is sufficient to intensify the olfactory impression of the fragrance(s).

10. Perfumed product containing one or more compound(s) of the formula (II), (II'), (III) or (IV) according to claim 1, a composition according to claim 2 or a fragrance mixture according to one or more of claims 4 to 8, in a sensorily effective quantity, wherein the product contains one or more surfactants.

11. A perfumed product according to claim 10, **characterized in that** the product is selected from the group consisting of detergents, cleaners, sanitary agents and toiletries for use in the field of body care, hair care, cosmetics or the household.

12. Method for endowing hair or textile fibers with one, several or all of the notes selected from the group consisting of fruity, floral, spicy, woody, musk and ambrette comprising the following steps:
(i) preparing a fragrance mixture according to any one of claims 4 to 8, wherein the (further) fragrance(s) not corresponding to the formula (II), (II'), (III) or (IV) mediate(s) one, several or all of the notes fruity, floral, spicy, woody, musk and ambrette and the quantity of the compound(s) of the forumla (II), (II'), (III) or (IV) is sufficient to modify and/or intensify one, several or all of the notes fruity, floral, spicy, woody, musk and ambrette, and
(ii) applying the fragrance mixture onto the hair or the textile fibers.

## Revendications

1. Composé de formule (II) ou (II') 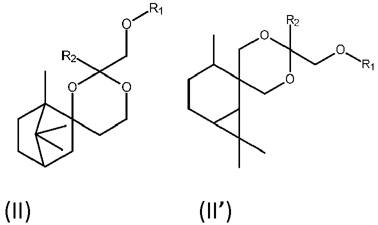 dans lequel R₁ est un hydrogène ou un groupe alkyle en C₁-C₃ et dans lequel R₂ est un hydrogène ou un groupe alkyle en C₁-C₄ ou le composé de formule 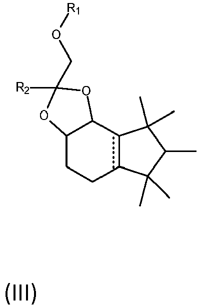 dans lequel R₁ est un hydrogène ou un groupe alkyle en C₁-C₃ et dans lequel R₂ est un hydrogène ou un groupe alkyle en C₁-C₄ et dans lequel le composé est saturé ou insaturé ou
de la formule (IV) ou (IV') 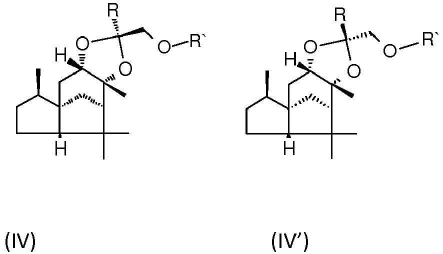 avec la condition: R = H ou le groupe alkyle en C₁-C₄ et R' = H ou le groupe alkyle en C₁-C₃.

2. Composition comprenant ou constituée de
(i) un ou plusieurs groupe(s) de 3,6,8,8-tétraméthyl-octahydro-3a,7-méthano-azulène-5,6-(diolketal(en)) et
(ii) un ou plusieurs composés de formule (II), (II'), (III), (IV) et/ou (IV') selon la revendication 1,
**caractérisée en ce que**
- la proportion totale des composés de formule (II), (II'), (III), (IV) et/ou (IV') est supérieure à 40 pour-cent en poids en termes de la quantité totale des 3,6,8,8-tétraméthyl-octahydro-3a,7-méthano-azulène-5,6-(diolketal(en)) contenus dans ladite composition,
et/ou **en ce que**
- le rapport pondéral entre le(s) composé(s) de formule (IV) et le(s) composé(s) de formule (IV'), s'il est présente, est dans la plage de 4 : 1 à 1 : 10.

3. Utilisation d'un composé de formule(II), (II'), (III) ou (IV) selon la revendication 1 ou selon une composition suivant la revendication 2 comme
- parfum ou mélange des parfums avec l'ambre et/ou le bois,
- moyen pour augmenter la texture et/ou la rétention d'un mélange des parfums, ou
- fixateur.

4. Mélange des parfums comprenant ou constitué de
- un ou plusieurs composé(s) de formule (II), (II'), (III) ou (IV) selon la revendication 1 ou une composition selon la revendication 2
ainsi que
- un ou plusieurs parfum(s) (de plus) pas correspondants à la formule (II), (II'), (III) ou (IV),
caractérisé(s) en ce que
(i) la quantité du(des) composé(s) de formule (II), (II'),(III) ou (IV) est suffisante pour transmettre un note d'ambre et/ou boisée,
et/ou que
(ii) le(s) parfum(s) pas correspondent(s) à la formule (II), (II'), (III) ou (IV) transmet(tent) une, plusieurs ou toutes les note(s) fruitée(s), florale(s), épicé(s), boisée(s), de musc ou ambrée(s) et que la quantité des composés de formule (II), (II'), (III) ou (IV) est suffisante pour modifier et/ou renforcer une, plusieurs ou toutes les note(s) fruitée(s), florale(s), épicée(s), boisée(s), de musc ou ambrée(s),
et/ou que
(iii) la quantité du(des) composé(s) de formule (II), (II'), (III) ou (IV) est suffisante pour donner un aspect volumineux, complexe, élégant et/ou naturel au mélange des parfums,
et/ou que
(iv) la quantité du(des) composé(s) de formule (II), (II'), (III) ou (IV) est suffisante pour transmettre une impression d'odeur plus nourrissante, harmonique, de plus grande qualité et/ou plus naturelle lors de la comparaison avec un mélange des parfums ayant une composition identique à l'exception du composé(s) de formule (II), (II'), (III) ou (IV).

5. Mélange de parfums selon la revendication 4 **caractérisée par** la quantité de composé(s) de formule (II), (II'), (III) ou (IV) dans la plage de 0,0001 à 25 pour-cent en poids concernant le poids total du mélange des parfums.

6. Mélange de parfums selon la revendication 4 ou 5 **caractérisée en ce que** le mélange de parfums
(i) contient un ou plusieurs parfum(s) boisé(s) et/ou ambré(s) de plus pas correspondants à la formule (II), (II'), (III) ou (IV)
(ii) contient un ou plusieurs parfum(s) de musc

7. Mélange de parfums selon la revendication 6 **caractérisée en ce que** le(s) parfum(s) de musc est/sont choisi(s) dans le groupe constitué par le parfum macrocyclique de musc, le parfum de musc Nitro, le parfum polycyclique de musc, le parfum alicyclique de musc ou les combinaisons de ceux-ci.

8. Mélange de parfums selon une ou plusieurs des revendication(s) 6 à 7 **caractérisée en ce que** le rapport pondéral entre des parfums de musc et du/des composé(s) de formule (II), (II'), (III) ou (IV) est compris dans une plage de 100 : 1 à 100.000 : 1.

9. Procédé de renforcement d'un parfum ou de plusieurs parfums choisi(s) dans le groupe constitué par le parfum fruité, florale, épicé, boisée, de musc ou ambré comprenant l'étape consistant à:
(i) Mélange d'un ou plusieurs parfums avec une, plusieurs ou toutes les notes choisie(s) dans le groupe constitué par le parfum fruité, florale, épicé, boisée, de musc ou ambré avec un ou plusieurs compose(s) de formule(II), (II'), (III) ou (IV) selon la revendication 1, une composition selon la revendication 2 ou un mélange de parfums selon une ou plusieurs des revendication(s) 4 à 8 dans une quantité suffisante pour renforcer l'impression d'odeur du/des parfum(s).

10. Le produit parfumé contenants un, plusieurs ou tous les composé(s) de formule (II), (II'), (III) ou (IV) selon la revendication 1, une composition selon la revendication 2 ou un mélange de parfums selon une ou plusieurs des revendication(s) 4 à 8 dans une quantité sensorielle efficace cependant le produit contient un ou plusieurs agents tensioactif(s).

11. Le produit parfumé selon la revendication 10 **caractérisée en ce que** le produit choisi dans le groupe constitué par les détergents, les nettoyants, les produits d'hygiène et les produits d'entretien pour l'application dans le domaine du soin corporel, du soin des cheveux, de la cosmétique ou du ménage.

12. Procédé pour ajouter une, plusieurs ou toutes les notes choisi(s) dans le groupe constitué par le parfum fruité, florale, épicé, boisé, de musc ou ambré aux cheveux ou aux fibres textiles comprenant les étapes consistants à:
(i) Fournir un mélange de parfums selon une des revendications 4 à 8, dans lequel un/plusieurs parfum(s) (de plus) pas correspondants à la formule (II), (II'), (III) ou (IV) transmet(tent) une, plusieurs ou toutes les notes fruitée, florale, épicée, boisée, de musc ou ambré et dans lequel la quantité du(des) composé(s) de formule (II), (II'), (III) ou (IV) est suffisante pour modifier et/ou renforcer une, plusieurs ou toutes les note(s) fruitée(s), florale(s), épicée(s), boisée(s), de musc ou ambrée(s), et
(ii) Appliquer du mélange de parfums aux cheveux ou aux fibres textiles.
